(19) Europäisches Patentamt
European Patent Office
Office européen des brevets

(11) EP 3 828 176 A1

(12) **EUROPEAN PATENT APPLICATION**
published in accordance with Art. 153(4) EPC

(43) Date of publication:
**02.06.2021 Bulletin 2021/22**

(21) Application number: **19841691.9**

(22) Date of filing: **23.07.2019**

(51) Int Cl.:
***C07D 261/12** (2006.01)* ***A01N 43/80** (2006.01)*
***A01P 13/00** (2006.01)*

(86) International application number:
**PCT/JP2019/028904**

(87) International publication number:
**WO 2020/022350 (30.01.2020 Gazette 2020/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **24.07.2018 JP 2018138495**

(71) Applicant: **Hokko Chemical Industry Co., Ltd.**
**Tokyo 103-8341 (JP)**

(72) Inventors:
- **SUZUKI, Jun**
**Atsugi-shi, Kanagawa 243-0023 (JP)**
- **KOYAMA, Kohei**
**Atsugi-shi, Kanagawa 243-0023 (JP)**

(74) Representative: **Reitstötter Kinzebach**
**Patentanwälte**
**Sternwartstrasse 4**
**81679 München (DE)**

(54) **ISOXAZOLIN-5-ONE DERIVATIVE, AND HERBICIDE CONTAINING SAME AS ACTIVE INGREDIENT**

(57) An isoxazolin-5-one derivative represented by formula (1) below:

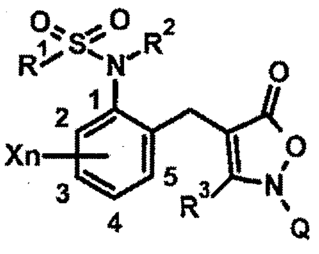

(1)

wherein $R^1$ represents a C1-C6 haloalkyl group, Q represents a C3-C8 cycloalkyl group substituted with a C1-C6 alkoxy group, each of $R^2$, $R^3$ and X represents a certain substituent or the like, and n represents an integer of 1 to 4, wherein X's may be different from each other when n represents an integer of 2 to 4, and a herbicide containing the isoxazolin-5-one derivative as an active ingredient are provided.

EP 3 828 176 A1

## Description

TECHNICAL FIELD

The present invention relates to isoxazolin-5-one derivatives and herbicides which contain the isoxazolin-5-one derivatives as active ingredients and which have a particularly excellent control effect on a harmful weed in an agricultural or horticultural field or in a non-crop land.

BACKGROUND ART

Use of herbicides is indispensable for protecting useful crops such as rice, wheat, corn, soybeans, cotton plant and beets from weeds and for increasing the yield. Recently, a selective herbicide which does not damage crops but which can selectively kill weeds only is desired in cultivated land where both such a useful crop and weeds grow. Moreover, an agent which exhibits a high herbicidal effect with a possible low agent amount is required in view of the prevention of environmental pollution, a decrease in the economic costs in transport and spraying and the like.

Here, although isoxazolin-5-one derivatives exhibiting a similar herbicidal activity to that of the invention are reported in Patent Literatures 1 and 2 and Non Patent Literature 1, the compounds of the invention in which the 4-position of the isoxazolin-5-one ring is substituted with a 2-(haloalkylsulfonylamino)benzyl group are not reported at all. It is known that heterocyclic compounds having a haloalkylsulfonylamino group at the 2-position of a benzyl group have a herbicidal activity (Patent Literatures 3 to 12). However, there is no report showing that compounds in which the heterocyclic moiety is isoxazolin-5-one, as in the invention, exhibit a herbicidal activity.

CITATION LIST

PATENT LITERATURE

Patent Literature 1: US Patent No. 4000155
Patent Literature 2: German Published Patent No. 3541722
Patent Literature 3: WO2004/011429
Patent Literature 4: WO2006/090792
Patent Literature 5: WO2008/059948
Patent Literature 6: WO2008/102908
Patent Literature 7: WO2010/026989
Patent Literature 8: WO2010/119906
Patent Literature 9: WO2014/175206
Patent Literature 10: WO2016/056565
Patent Literature 11: WO2015/004282
Patent Literature 12: WO2015/097071


NON PATENT LITERATURE

Non Patent Literature 1: Journal of Heterocyclic Chemistry, Vol. 50, 2013, P.1381-1385

SUMMARY OF INVENTION

TECHNICAL PROBLEM

A problem to be solved by the invention is to provide a herbicide having an excellent herbicidal activity and crop selectivity.

SOLUTION TO PROBLEM

As a result of intensive studies to solve the problem, the present inventors have found that isoxazolin-5-one derivatives represented by formula (1) below exhibit an excellent herbicidal activity and thus have accomplished the invention.

Accordingly, the first invention of the present application relates to isoxazolin-5-one derivatives represented by

formula (1) below (sometimes referred to as "the compounds of the invention" in this description).

The second invention of the application relates to herbicides characterized by containing an isoxazolin-5-one derivative represented by formula (1) as an active ingredient.

Namely, the inventors have found that the following constitutions can solve the problem.

[1] An isoxazolin-5-one derivative represented by formula (1) below:

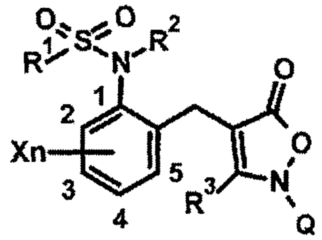

(1)

wherein $R^1$ represents a C1-C6 haloalkyl group,

$R^2$ represents a hydrogen atom, a C1-C6 alkyl group, a C1-C6 haloalkyl group, a C2-C6 alkenyl group, a C2-C6 alkynyl group, a C1-C6 alkoxy C1-C6 alkyl group, a C1-C6 haloalkoxy C1-C6 alkyl group, a C1-C6 alkoxy C1-C6 alkoxy C1-C6 alkyl group, a C1-C6 alkylthio C1-C6 alkyl group, a C1-C6 alkylcarbonyl C1-C6 alkyl group, a C7-C11 aralkyl group (the group may be monosubstituted or polysubstituted with a halogen atom(s), a C1-C6 alkyl group(s) or a C1-C6 alkoxy group(s)), a phenoxy C1-C6 alkyl group, a C7-C11 aralkyloxy C1-C6 alkyl group, a phenylcarbonyl C1-C6 alkyl group, a C1-C6 alkylcarbonyl group, a C1-C6 haloalkylcarbonyl group, a C2-C6 alkenylcarbonyl group, a C2-C6 alkynylcarbonyl group, a C3-C6 cycloalkylcarbonyl group, a C3-C6 cycloalkyl C1-C6 alkylcarbonyl group, a C1-C6 alkoxy C1-C6 alkylcarbonyl group, a C1-C6 haloalkoxy C1-C6 alkylcarbonyl group, a C1-C6 alkoxy C1-C6 alkoxy C1-C6 alkylcarbonyl group, a C1-C6 alkylthio C1-C6 alkylcarbonyl group, a C1-C6 haloalkylthio C1-C6 alkylcarbonyl group, a benzoyl group (the group may be monosubstituted or polysubstituted with a halogen atom(s) or a C1-C6 alkyl group(s)), a C7-C11 aralkylcarbonyl group (the group may be monosubstituted or polysubstituted with a halogen atom(s) or a C1-C6 alkyl group(s)), a heterocyclic carbonyl group (the group may be monosubstituted or polysubstituted with a halogen atom(s) or a C1-C6 alkyl group(s)), a heterocyclic C1-C6 alkylcarbonyl group (the group may be monosubstituted or polysubstituted with a halogen atom(s) or a C1-C6 alkyl group(s)), a C1-C6 alkoxycarbonyl group, a C1-C6 haloalkoxycarbonyl group, a C2-C6 alkenyloxycarbonyl group, a C2-C6 alkynyloxycarbonyl group, a C3-C6 cycloalkyloxycarbonyl group, a C3-C6 cycloalkyl C1-C6 alkoxycarbonyl group, a C1-C6 alkoxy C1-C6 alkoxycarbonyl group, a C1-C6 haloalkoxy C1-C6 alkoxycarbonyl group, a C1-C6 alkoxy C1-C6 alkoxy C1-C6 alkoxycarbonyl group, a C1-C6 alkylthio C1-C6 alkoxycarbonyl group, a C1-C6 haloalkylthio C1-C6 alkoxycarbonyl group, a phenoxycarbonyl group (the group may be monosubstituted or polysubstituted with a halogen atom(s) or a C1-C6 alkyl group(s)), a C7-C11 aralkyloxycarbonyl group (the group may be monosubstituted or polysubstituted with a halogen atom(s) or a C1-C6 alkyl group(s)), a phenoxy C1-C6 alkoxycarbonyl group (the group may be monosubstituted or polysubstituted with a halogen atom(s) or a C1-C6 alkyl group(s)), a heterocyclic oxycarbonyl group (the group may be monosubstituted or polysubstituted with a halogen atom(s) or a C1-C6 alkyl group(s)), a heterocyclic C1-C6 alkoxycarbonyl group (the group may be monosubstituted or polysubstituted with a halogen atom(s) or a C1-C6 alkyl group(s)), a C1-C6 alkylthiocarbonyl group, a C1-C6 haloalkylthiocarbonyl group, a C1-C6 alkylaminocarbonyl group, a C1-C6 haloalkylaminocarbonyl group, a di-C1-C6 alkylaminocarbonyl group (the di-C1-C6 alkyl group moieties in the group may be the same or different), a C1-C6 alkylsulfonyl group, a C1-C6 haloalkylsulfonyl group, a C2-C6 alkenylsulfonyl group, a C2-C6 alkynylsulfonyl group, a C3-C6 cycloalkylsulfonyl group, a C3-C6 cycloalkyl C1-C6 alkylsulfonyl group, a C1-C6 alkoxy C1-C6 alkylsulfonyl group, a phenylsulfonyl group (the group may be monosubstituted or polysubstituted with a halogen atom(s) or a C1-C6 alkyl group(s)), a C7-C11 aralkylsulfonyl group (the group may be monosubstituted or polysubstituted with a halogen atom(s) or a C1-C6 alkyl group(s)), a C1-C6 alkylaminosulfonyl group or a di-C1-C6 alkylaminosulfonyl group (the di-C1-C6 alkyl group moieties in the group may be the same or different),

$R^3$ represents a hydrogen atom, a halogen atom, a C1-C6 alkyl group, a C1-C6 haloalkyl group, a C3-C6 cycloalkyl group, a phenyl group (the group may be monosubstituted or polysubstituted with a halogen atom(s) or a C1-C6 alkyl group(s)), an amino group, a C1-C6 alkylamino group or a di-C1-C6 alkylamino group (the di-C1-C6 alkyl group moieties in the group may be the same or different),

Q represents a C3-C8 cycloalkyl group substituted with a C1-C6 alkoxy group,

X represents a hydrogen atom, a halogen atom, a C1-C6 alkyl group or a C1-C6 alkoxy group, and

n represents an integer of 1 to 4, wherein X's may be different from each other when n represents an integer of 2 to 4.

[2] The isoxazolin-5-one derivative according to [1], wherein $R^1$ in formula (1) represents a C1-C6 fluoroalkyl group.
[3] The isoxazolin-5-one derivative according to [1] or [2], wherein $R^1$ in formula (1) represents a trifluoromethyl group.
[4] A herbicide containing the isoxazolin-5-one derivative according to any one of [1] to [3] as an active ingredient.

EFFECTS OF INVENTION

The novel isoxazolin-5-one derivatives of the invention represented by formula (1) above exhibit an excellent herbicide effect.

DESCRIPTION OF EMBODIMENTS

The isoxazolin-5-one derivatives related to the compounds of the invention, production methods thereof and the herbicides containing the compounds as active ingredients are explained specifically.

In the groups below, the carbon atom numbers described in groups containing carbonyl, such as a C1-C6 alkylcarbonyl group and a C1-C6 alkoxycarbonyl group, do not include the carbonyl carbon atom(s).

Additionally, when a C7-C11 aralkyl group has a substituent(s), the carbon atom numbers of the aralkyl group do not include the number of the carbon atoms contained in the substituent(s).

When groups containing an aralkyl group(s), such as a C7-C11 aralkylcarbonyl group, a C7-C11 aralkyloxycarbonyl group and a C7-C11 aralkylsulfonyl group, have a substituent(s), the carbon atom numbers of the aralkyl group(s) in the groups containing the aralkyl group(s) do not include the number of the carbon atoms contained in the substituent(s).

When groups containing an alkyl group(s), such as a heterocyclic C1-C6 alkylcarbonyl group, a phenoxy C1-C6 alkoxycarbonyl group and a heterocyclic C1-C6 alkoxycarbonyl group, have a substituent(s), the carbon atom numbers of the alkyl group(s) in the groups containing the alkyl group(s) do not include the number of the carbon atoms contained in the substituent(s).

In the isoxazolin-5-one derivatives represented by formula (1) of the invention, examples of the halogen atom or the halogen atom as a substituent include elements of fluorine, chlorine, bromine or iodine. The number of the halogen atom(s) as a substituent may be one, two or more, and when the number is two or more, the halogen atoms may be the same or different from each other. The position of substitution with the halogen atom(s) may be any position.

Examples of the C1-C6 haloalkyl group represented by $R^1$, $R^2$ or $R^3$ include a monofluoromethyl group, a difluoromethyl group, a trifluoromethyl group, a 2,2,2-trifluoroethyl group, a monochloromethyl group, a 2-chloroethyl group, a trichloromethyl group, a 1-fluoroethyl group, a 2-fluoroethyl group, a 6-fluorohexyl group and the like.

Examples of the C1-C6 alkyl group represented by $R^2$, $R^3$ or X or the C1-C6 alkyl group as a substituent include a methyl group, an ethyl group, an n-propyl group, an isopropyl group, an n-butyl group, an isobutyl group, a sec-butyl group, a tert-butyl group, an n-pentyl group, a neopentyl group, a 2-pentyl group, a 3-pentyl group, a tert-pentyl group, an n-hexyl group, an isohexyl group, a 2-hexyl group, a 3-hexyl group and the like. The number of the C1-C6 alkyl group(s) as a substituent may be one, two or more, and when the number is two or more, the C1-C6 alkyl groups may be the same or different from each other. The position of substitution with the C1-C6 alkyl group maybe any position.

Examples of the C2-C6 alkenyl group represented by $R^2$ include vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group and the like.

Examples of the C2-C6 alkynyl group represented by $R^2$ include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group and the like.

Examples of the C1-C6 alkoxy C1-C6 alkyl group represented by $R^2$ include a methoxymethyl group, an ethoxymethyl group, an n-propoxymethyl group, an isopropoxymethyl group, an n-butoxymethyl group, a sec-butoxymethyl group, a tert-butoxymethyl group, a 1-pentyloxymethyl group, a 1-hexyloxymethyl group, a 2-methoxyethyl group, a 2-ethoxyethyl group, a 2-isopropoxyethyl group, a 2-isobutoxyethyl group, a 3-methoxypropyl group, a 2-methoxypropyl group, a 2-methoxy-1-methylethyl group and the like.

Examples of the C1-C6 haloalkoxy C1-C6 alkyl group represented by $R^2$ include a trifluoromethoxymethyl group, a 2,2,2-trifluoroethoxymethyl group, a 2-(2,2,2-trifluoroethoxy)ethyl group and the like.

Examples of the C1-C6 alkoxy C1-C6 alkoxy C1-C6 alkyl group represented by $R^2$ include a methoxyethoxymethyl group, an ethoxyethoxymethyl group, a methoxyethoxyethyl group, an ethoxyethoxyethyl group and the like.

Examples of the C1-C6 alkylthio C1-C6 alkyl group represented by $R^2$ include a methylthiomethyl group, an ethylthiomethyl group, an n-propylthiomethyl group, an isopropylthiomethyl group, an n-butylthiomethyl group, a sec-butylthiomethyl group, a tert-butylthiomethyl group, a 1-pentylthiomethyl group, a 1-hexylthiomethyl group, a 2-methylthioethyl group, a 2-ethylthioethyl group, a 2-isopropylthioethyl group, a 2-isobutylthioethyl group, a 3-methylthiopropyl group, a 2-methylthiopropyl group, a 2-methylthio-1-methylethyl group, a 2-methylthio-1-methylpropyl group and the like.

Examples of the C1-C6 alkylcarbonyl C1-C6 alkyl group represented by $R^2$ include a 2-oxopropyl group, a 2-oxobutyl group, a 3-oxobutyl group and the like.

Examples of the C7-C11 aralkyl group represented by $R^2$ include a benzyl group, a 1-phenethyl group, a 2-phenethyl group, a 1-phenylpropyl group, a 2-phenylpropyl group, a 3-phenylpropyl group, a 1-phenyl-2-methylpropyl group, a 1-phenylbutyl group, a 1-phenylpentyl group and the like.

The C7-C11 aralkyl group may be monosubstituted or polysubstituted with a halogen atom(s), a C1-C6 alkyl group(s) or a C1-C6 alkoxy group(s).

Examples of the phenoxy C1-C6 alkyl group represented by $R^2$ include a 2-phenoxyethyl group, a 2-phenoxypropyl group, a 3-phenoxypropyl group, a 2-phenoxybutyl group, a 3-phenoxybutyl group, a 4-phenoxybutyl group and the like.

Examples of the C7-C11 aralkyloxy C1-C6 alkyl group represented by $R^2$ include a benzyloxymethyl group, a 1-phenethyloxymethyl group, a 2-phenethyloxymethyl group, a 1-phenylpropoxymethyl group, a 2-phenylpropoxymethyl group, a 3-phenylpropoxymethyl group, a benzyloxyethyl group and the like.

Examples of the phenylcarbonyl C1-C6 alkyl group represented by $R^2$ include a phenacyl group, a 1-phenyl-1-oxopropyl group, a 1-phenyl-2-oxopropyl group and the like.

Examples of the C1-C6 alkylcarbonyl group represented by $R^2$ include an acetyl group, an ethylcarbonyl group, an n-propylcarbonyl group, an isopropylcarbonyl group, an n-butylcarbonyl group, an isobutylcarbonyl group, a sec-butylcarbonyl group, a tert-butylcarbonyl group, a 1-pentylcarbonyl group, a 1-hexylcarbonyl group and the like.

Examples of the C1-C6 haloalkylcarbonyl group represented by $R^2$ include a monofluoromethylcarbonyl group, a difluoromethylcarbonyl group, a trifluoromethylcarbonyl group, a 2,2,2-trifluoroethylcarbonyl group, a 2-chloroethylcarbonyl group, a trichloromethylcarbonyl group, a 1-fluoroethylcarbonyl group, a 2-fluoroethylcarbonyl group, a 6-fluorohexylcarbonyl group and the like.

Examples of the C2-C6 alkenylcarbonyl group represented by $R^2$ include an acryloyl group, a methacryloyl group and the like.

Examples of the C2-C6 alkynylcarbonyl group represented by $R^2$ include a propiolyl group, a methylpropiolyl group and the like.

Examples of the C3-C6 cycloalkylcarbonyl group represented by $R^2$ include a cyclopropanecarbonyl group, a 1-methylcyclopropanecarbonyl group, a 2-methylcyclopropanecarbonyl group, a 2,2-dimethylcyclopropanecarbonyl group, a cyclobutanecarbonyl group, a cyclopentanecarbonyl group, a cyclohexanecarbonyl group and the like.

Examples of the C3-C6 cycloalkyl C1-C6 alkylcarbonyl group represented by $R^2$ include a cyclopropylmethylcarbonyl group, a cyclopropylethylcarbonyl group, a 1-methylcyclopropylmethylcarbonyl group, a 2-methylcyclopropylmethylcarbonyl group, a 2,2-dimethylcyclopropylmethylcarbonyl group, a cyclobutylmethylcarbonyl group, a cyclopentylmethylcarbonyl group, a cyclohexylmethylcarbonyl group and the like.

Examples of the C1-C6 alkoxy C1-C6 alkylcarbonyl group represented by $R^2$ include a methoxymethylcarbonyl group, an ethoxymethylcarbonyl group, an n-propoxymethylcarbonyl group, an isopropoxymethylcarbonyl group, an n-butoxymethylcarbonyl group, a sec-butoxymethylcarbonyl group, a tert-butoxymethylcarbonyl group, a 1 -pentyloxymethylcarbonyl group, a 1-hexyloxymethylcarbonyl group, a 2-methoxyethylcarbonyl group, a 2-ethoxyethylcarbonyl group, a 2-isopropoxyethylcarbonyl group, a 2-isobutoxyethylcarbonyl group, a 3-methoxypropylcarbonyl group, a 2-methoxypropylcarbonyl group, a 2-methoxy-1-methylethylcarbonyl group and the like.

Examples of the C1-C6 haloalkoxy C1-C6 alkylcarbonyl group represented by $R^2$ include a trifluoromethoxymethylcarbonyl group, a 2,2,2-trifluoroethoxymethylcarbonyl group, a 2-(2,2,2-trifluoroethoxy)ethylcarbonyl group and the like.

Examples of the C1-C6 alkoxy C1-C6 alkoxy C1-C6 alkylcarbonyl group represented by $R^2$ include a methoxyethoxymethylcarbonyl group, an ethoxyethoxymethylcarbonyl group, a methoxyethoxyethylcarbonyl group, an ethoxyethoxyethylcarbonyl group and the like.

Examples of the C1-C6 alkylthio C1-C6 alkylcarbonyl group represented by $R^2$ include a methylthiomethylcar-

bonyl group, an ethylthiomethylcarbonyl group, an n-propylthiomethylcarbonyl group, an isopropylthiomethylcarbonyl group, an n-butylthiomethylcarbonyl group, a sec-butylthiomethylcarbonyl group, a tert-butylthiomethylcarbonyl group, a 1-pentylthiomethylcarbonyl group, a 1-hexylthiomethylcarbonyl group, a 2-methylthioethylcarbonyl group, a 2-ethylthioethylcarbonyl group, a 2-isopropylthioethylcarbonyl group, a 2-isobutylthioethylcarbonyl group, a 3-methylthiopropylcarbonyl group, a 2-methylthiopropylcarbonyl group, a 2-methylthio-1-methylethylcarbonyl group, a 2-methylthio-1-methylpropylcarbonyl group and the like.

Examples of the C1-C6 haloalkylthio C1-C6 alkylcarbonyl group represented by $R^2$ include a monofluoromethylthiomethylcarbonyl group, a difluoromethylthiomethylcarbonyl group, a trifluoromethylthiomethylcarbonyl group, a 2,2,2-trifluoroethylthiomethylcarbonyl group, a 2-chloroethylthiomethylcarbonyl group, a trichloromethylthiomethylcarbonyl group, a 1-fluoroethylthiomethylcarbonyl group, a 2-fluoroethylthiomethylcarbonyl group, a 6-fluorohexylthiomethylcarbonyl group and the like.

The benzoyl group represented by $R^2$ may be monosubstituted or polysubstituted with a halogen atom(s) or a C1-C6 alkyl group(s).

Examples of the C7-C11 aralkylcarbonyl group represented by $R^2$ include a benzylcarbonyl group, a 1-phenethylcarbonyl group, a 2-phenethylcarbonyl group, a 1-phenylpropylcarbonyl group, a 2-phenylpropylcarbonyl group, a 3-phenylpropylcarbonyl group, a 1-phenyl-2-methylpropylcarbonyl group, a 1-phenylbutylcarbonyl group, a 1-phenylpentylcarbonyl group and the like.

The C7-C11 aralkylcarbonyl group may be monosubstituted or polysubstituted with a halogen atom(s) or a C1-C6 alkyl group(s).

Examples of the heterocyclic carbonyl group represented by $R^2$ include a 2-pyridylcarbonyl group, a 3-pyridylcarbonyl group, a 4-pyridylcarbonyl group, a 2-thienylcarbonyl group, a 3-thienylcarbonyl group, a 2-tetrahydrofurylcarbonyl group, a 3-tetrahydrofurylcarbonyl group and the like.

The heterocyclic carbonyl group may be monosubstituted or polysubstituted with a halogen atom(s) or a C1-C6 alkyl group(s).

Examples of the heterocyclic C1-C6 alkylcarbonyl group represented by $R^2$ include a 2-pyridylmethylcarbonyl group, a 3-pyridylmethylcarbonyl group, a 4-pyridylmethylcarbonyl group, a 2-thienylmethylcarbonyl group, a 3-thienylmethylcarbonyl group, a 2-tetrahydrofurfurylcarbonyl group, a 3-tetrahydrofurfurylcarbonyl group and the like.

The heterocyclic C1-C6 alkylcarbonyl group may be monosubstituted or polysubstituted with a halogen atom(s) or a C1-C6 alkyl group(s).

Examples of the C1-C6 alkoxycarbonyl group represented by $R^2$ include a methoxycarbonyl group, an ethoxycarbonyl group, an n-propoxycarbonyl group, an isopropoxycarbonyl group, an n-butoxycarbonyl group, an isobutoxycarbonyl group, a sec-butoxycarbonyl group, a tert-butoxycarbonyl group, an n-pentyloxycarbonyl group, a neopentyloxycarbonyl group, a 2-pentyloxycarbonyl group, a 3-pentyloxycarbonyl group, an n-hexyloxycarbonyl group and the like.

Examples of the C1-C6 haloalkoxycarbonyl group represented by $R^2$ include a trifluoromethoxycarbonyl group, a 2,2,2-trifluoroethoxycarbonyl group and the like.

Examples of the C2-C6 alkenyloxycarbonyl group represented by $R^2$ include a vinyloxycarbonyl group, a 1-propenyloxycarbonyl group, a 2-propenyloxycarbonyl group, a 1-butenyloxycarbonyl group, a 2-butenyloxycarbonyl group, a 3-butenyloxycarbonyl group and the like.

Examples of the C2-C6 alkynyloxycarbonyl group represented by $R^2$ include an ethynyloxycarbonyl group, a 1-propynyloxycarbonyl group, a propargyloxycarbonyl group, a 1-butynyloxycarbonyl group, a 2-butynyloxycarbonyl group, a 3-butynyloxycarbonyl group, a 1-methyl-2-propynyloxycarbonyl group, a 2-methyl-3-butynyloxycarbonyl group and the like.

Examples of the C3-C6 cycloalkyloxycarbonyl group represented by $R^2$ include a cyclopropyloxycarbonyl group, a 1-methylcyclopropyloxycarbonyl group, a 2-methylcyclopropyloxycarbonyl group, a 2,2-dimethylcyclopropyloxycarbonyl group, a cyclobutyloxycarbonyl group, a cyclopentyloxycarbonyl group, a cyclohexyloxycarbonyl group and the like.

Examples of the C3-C6 cycloalkyl C1-C6 alkoxycarbonyl group represented by $R^2$ include a cyclopropylmethoxycarbonyl group, a 1-methylcyclopropylmethoxycarbonyl group, a 2-methylcyclopropylmethoxycarbonyl group, a 2,2-dimethylcyclopropylmethoxycarbonyl group, a cyclobutylmethoxycarbonyl group, a cyclopentylmethoxycarbonyl group, a cyclohexylmethoxycarbonyl group and the like.

Examples of the C1-C6 alkoxy C1-C6 alkoxycarbonyl group represented by $R^2$ include a methoxymethoxycarbonyl group, an ethoxymethoxycarbonyl group, an n-propoxymethoxycarbonyl group, an isopropoxymethoxycarbonyl group, an n-butoxymethoxycarbonyl group, a sec-butoxymethoxycarbonyl group, a tert-butoxymethoxycarbonyl group, a 1-pentyloxymethoxycarbonyl group, a 1-hexyloxymethoxycarbonyl group, a 2-methoxyethoxycarbonyl group, a 2-ethoxyethoxycarbonyl group, a 2-isopropoxyethoxycarbonyl group, a 2-isobutoxyethoxycarbonyl group, a 3-methoxypropoxycarbonyl group, a 2-methoxypropoxycarbonyl group, a 2-methoxy-1-methylethoxycarbonyl group and the like.

Examples of the C1-C6 haloalkoxy C1-C6 alkoxycarbonyl group represented by $R^2$ include a trifluoromethoxymethoxycarbonyl group, a 2,2,2-trifluoroethoxymethoxycarbonyl group, a 2-(2,2,2-trifluoroethoxy)ethoxycarbonyl group and the like.

Examples of the C1-C6 alkoxy C1-C6 alkoxy C1-C6 alkoxycarbonyl group represented by $R^2$ include a methoxyethoxymethoxycarbonyl group, an ethoxyethoxymethoxycarbonyl group, a methoxyethoxyethoxycarbonyl group, an ethoxyethoxyethoxycarbonyl group and the like.

Examples of the C1-C6 alkylthio C1-C6 alkoxycarbonyl group represented by $R^2$ include a methylthiomethoxycarbonyl group, an ethylthiomethoxycarbonyl group, an n-propylthiomethoxycarbonyl group, an isopropylthiomethoxycarbonyl group, an n-butylthiomethoxycarbonyl group, a sec-butylthiomethoxycarbonyl group, a tert-butylthiomethoxycarbonyl group, a 1-pentylthiomethoxycarbonyl group, a 1-hexylthiomethoxycarbonyl group, a 2-methylthioethoxycarbonyl group, a 2-ethylthioethoxycarbonyl group, a 2-isopropylthioethoxycarbonyl group, a 2-isobutylthioethoxycarbonyl group, a 3-methylthiopropoxycarbonyl group, a 2-methylthiopropoxycarbonyl group, a 2-methylthio-1-methylethoxycarbonyl group, a 2-methylthio-1-methylpropoxycarbonyl group and the like.

Examples of the C1-C6 haloalkylthio C1-C6 alkoxycarbonyl group represented by $R^2$ include a monofluoromethylthiomethoxycarbonyl group, a difluoromethylthiomethoxycarbonyl group, a trifluoromethylthiomethoxycarbonyl group, a 2,2,2-trifluoroethylthiomethoxycarbonyl group, a 2-(2,2,2-trifluoroethylthio)ethoxycarbonyl group, a 2-chloroethylthiomethoxycarbonyl group, a trichloromethylthiomethoxycarbonyl group, a 1-fluoroethylthiomethoxycarbonyl group, a 2-fluoroethylthiomethoxycarbonyl group, a 6-fluorohexylthiomethoxycarbonyl group and the like.

The phenoxycarbonyl group represented by $R^2$ may be monosubstituted or polysubstituted with a halogen atom(s) or a C1-C6 alkyl group(s).

Examples of the C7-C11 aralkyloxycarbonyl group represented by $R^2$ include a benzyloxycarbonyl group, a 1-phenethyloxycarbonyl group, a 2-phenethyloxycarbonyl group, a 1-phenylpropoxycarbonyl group, a 2-phenylpropoxycarbonyl group, a 3-phenylpropoxycarbonyl group, a 1-phenyl-2-methylpropoxycarbonyl group, a 1-phenylbutoxycarbonyl group, a 1-phenylpentyloxycarbonyl group and the like.

The C7-C11 aralkyloxycarbonyl group may be monosubstituted or polysubstituted with a halogen atom(s) or a C1-C6 alkyl group(s).

Examples of the phenoxy C1-C6 alkoxycarbonyl group represented by $R^2$ include a 2-phenoxyethoxycarbonyl group, a 2-phenoxypropoxycarbonyl group, a 3-phenoxypropoxycarbonyl group, a 2-phenoxybutoxycarbonyl group, a 3-phenoxybutoxycarbonyl group, a 4-phenoxybutoxycarbonyl group and the like.

The phenoxy C1-C6 alkoxycarbonyl group may be monosubstituted or polysubstituted with a halogen atom(s) or a C1-C6 alkyl group(s).

Examples of the heterocyclic oxycarbonyl group represented by $R^2$ include a 2-pyridyloxycarbonyl group, a 3-pyridyloxycarbonyl group, a 4-pyridyloxycarbonyl group, a 2-thienyloxycarbonyl group, a 3-thienyloxycarbonyl group, a 2-tetrahydrofuryloxycarbonyl group, a 3-tetrahydrofuryloxycarbonyl group and the like.

The heterocyclic oxycarbonyl group may be monosubstituted or polysubstituted with a halogen atom(s) or a C1-C6 alkyl group(s).

Examples of the heterocyclic C1-C6 alkoxycarbonyl group represented by $R^2$ include a 2-pyridylmethyloxycarbonyl group, a 3-pyridylmethyloxycarbonyl group, a 4-pyridylmethyloxycarbonyl group, a 2-thienylmethyloxycarbonyl group, a 3-thienylmethyloxycarbonyl group, a 2-tetrahydrofurfuryloxycarbonyl group, a 3-tetrahydrofurfuryloxycarbonyl group and the like.

The heterocyclic C1-C6 alkoxycarbonyl group may be monosubstituted or polysubstituted with a halogen atom(s) or a C1-C6 alkyl group(s).

Examples of the C1-C6 alkylthiocarbonyl group represented by $R^2$ include a methylthiocarbonyl group, an ethylthiocarbonyl group, an n-propylthiocarbonyl group, an isopropylthiocarbonyl group, an n-butylthiocarbonyl group, an isobutylthiocarbonyl group, a sec-butylthiocarbonyl group, a tert-butylthiocarbonyl group and the like.

Examples of the C1-C6 haloalkylthiocarbonyl group represented by $R^2$ include a trifluoromethylthiocarbonyl group, a 2,2,2-trifluoroethylthiocarbonyl group and the like.

Examples of the C1-C6 alkylaminocarbonyl group represented by $R^2$ include a methylaminocarbonyl group, an ethylaminocarbonyl group, an n-propylaminocarbonyl group, an isopropylaminocarbonyl group, an n-butylaminocarbonyl group, an isobutylaminocarbonyl group, a sec-butylaminocarbonyl group, a tert-butylaminocarbonyl group and the like.

Examples of the C1-C6 haloalkylaminocarbonyl group represented by $R^2$ include a trifluoromethylaminocarbonyl group, a 2,2,2-trifluoroethylaminocarbonyl group and the like.

Examples of the di-C1-C6 alkylaminocarbonyl group represented by $R^2$ include a dimethylaminocarbonyl group, a methylethylaminocarbonyl group, a diethylaminocarbonyl group, a di-n-propylaminocarbonyl group, a methyl n-propylaminocarbonyl group, an ethyl n-propylaminocarbonyl group, a diisopropylaminocarbonyl group, a di-n-butylaminocarbonyl group, a diisobutylaminocarbonyl group, a di-sec-butylaminocarbonyl group, a di-tert-butylaminocarbonyl group and the like.

The di-C1-C6 alkyl group moieties of the di-C1-C6 alkylaminocarbonyl group may be the same or different.

Examples of the C1-C6 alkylsulfonyl group represented by $R^2$ include a methanesulfonyl group, an ethanesulfonyl group, an n-propanesulfonyl group, an isopropanesulfonyl group, an n-butanesulfonyl group, an isobutanesulfonyl

group, a sec-butanesulfonyl group, a tert-butanesulfonyl group, an n-pentanesulfonyl group and the like.

Examples of the C1-C6 haloalkylsulfonyl group represented by $R^2$ include a monofluoromethylsulfonyl group, a difluoromethylsulfonyl group, a trifluoromethylsulfonyl group, a monochloromethylsulfonyl group, a trichloromethylsulfonyl group, a 2,2,2-trifluoroethylsulfonyl group and the like.

Examples of the C2-C6 alkenylsulfonyl group represented by $R^2$ include a vinylsulfonyl group, a 1-propenylsulfonyl group, a 2-propenylsulfonyl group, a 1-butenylsulfonyl group, a 2-butenylsulfonyl group, a 3-butenylsulfonyl group and the like.

Examples of the C2-C6 alkynylsulfonyl group represented by $R^2$ include an ethynylsulfonyl group, a 1-propynylsulfonyl group, a propargylsulfonyl group, a 1-butynylsulfonyl group, a 2-butynylsulfonyl group, a 3-butynylsulfonyl group, a 1-methyl-2-propynylsulfonyl group, a 2-methyl-3-butynylsulfonyl group and the like.

Examples of the C3-C6 cycloalkylsulfonyl group represented by $R^2$ include a cyclopropanesulfonyl group, a 1-methylcyclopropanesulfonyl group, a 2-methylcyclopropanesulfonyl group, a 2,2-dimethylpropanesulfonyl group, a cyclobutanesulfonyl group, a cyclopentanesulfonyl group, a cyclohexanesulfonyl group and the like.

Examples of the C3-C6 cycloalkyl C1-C6 alkylsulfonyl group represented by $R^2$ include a cyclopropylmethylsulfonyl group, a 1-methylcyclopropylmethylsulfonyl group, a 2-methylcyclopropylmethylsulfonyl group, a 2,2-dimethylpropylmethylsulfonyl group, a cyclobutylmethylsulfonyl group, a cyclopentylmethylsulfonyl group, a cyclohexylmethylsulfonyl group and the like.

Examples of the C1-C6 alkoxy C1-C6 alkylsulfonyl group represented by $R^2$ include a methoxymethylsulfonyl group, an ethoxymethylsulfonyl group, an n-propoxymethylsulfonyl group, an isopropoxymethylsulfonyl group, an n-butoxymethylsulfonyl group, a sec-butoxymethylsulfonyl group, a tert-butoxymethylsulfonyl group, a 1-pentyloxymethylsulfonyl group, a 1-hexyloxymethylsulfonyl group, a 2-methoxyethylsulfonyl group, a 2-ethoxyethylsulfonyl group, a 2-isopropoxyethylsulfonyl group, a 2-isobutoxyethylsulfonyl group, a 3-methoxypropylsulfonyl group, a 2-methoxypropylsulfonyl group, a 2-methoxy-1-methylethylsulfonyl group and the like.

The phenylsulfonyl group represented by $R^2$ may be monosubstituted or polysubstituted with a halogen atom(s) or a C1-C6 alkyl group(s).

Examples of the C7-C11 aralkylsulfonyl group represented by $R^2$ include a benzylsulfonyl group, a 1-phenethylsulfonyl group, a 2-phenethylsulfonyl group, a 1-phenylpropylsulfonyl group, a 2-phenylpropylsulfonyl group, a 3-phenylpropylsulfonyl group, a 1-phenyl-2-methylpropylsulfonyl group, a 1-phenylbutylsulfonyl group, a 1-phenylpentylsulfonyl group and the like.

The C7-C11 aralkylsulfonyl group may be monosubstituted or polysubstituted with a halogen atom(s) or a C1-C6 alkyl group(s).

Examples of the C1-C6 alkylaminosulfonyl group represented by $R^2$ include a methylaminosulfonyl group, an ethylaminosulfonyl group, an n-propylaminosulfonyl group, an isopropylaminosulfonyl group, an n-butylaminosulfonyl group, an isobutylaminosulfonyl group, a sec-butylaminosulfonyl group, a tert-butylaminosulfonyl group and the like.

Examples of the di-C1-C6 alkylaminosulfonyl group represented by $R^2$ include a dimethylaminosulfonyl group, a methylethylaminosulfonyl group, a diethylaminosulfonyl group, a di-n-propylaminosulfonyl group, a methyl n-propylaminosulfonyl group, an ethyl n-propylaminosulfonyl group, a diisopropylaminosulfonyl group, a di-n-butylaminosulfonyl group, a diisobutylaminosulfonyl group, a di-sec-butylaminosulfonyl group, a di-tert-butylaminosulfonyl group and the like.

The di-C1-C6 alkyl group moieties of the di-C1-C6 alkylaminosulfonyl group may be the same or different.

Examples of the C3-C6 cycloalkyl group represented by $R^3$ include a cyclopropyl group, a 1-methylcyclopropyl group, a 2-methylcyclopropyl group, a 2,2-dimethylpropyl group, a cyclobutyl group, a cyclopentyl group, a cyclohexyl group and the like.

The phenyl group represented by $R^3$ may be monosubstituted or polysubstituted with a halogen atom(s) or a C1-C6 alkyl group(s).

Examples of the C1-C6 alkylamino group represented by $R^3$ include a methylamino group, an ethylamino group, an n-propylamino group, an isopropylamino group, an n-butylamino group, an isobutylamino group, a sec-butylamino group, a tert-butylamino group and the like.

Examples of the di-C1-C6 alkylamino group represented by $R^3$ include a dimethylamino group, a methylethylamino group, a diethylamino group, a di-n-propylamino group, a methyl n-propylamino group, an ethyl n-propylamino group, a diisopropylamino group, a di-n-butylamino group, a diisobutylamino group, a di-sec-butylamino group, a di-tert-butylamino group and the like.

The di-C1-C6 alkyl group moieties of the di-C1-C6 alkylamino group may be the same or different.

Examples of the C3-C8 cycloalkyl group moiety of the C3-C8 cycloalkyl group substituted with a C1-C6 alkoxy group represented by Q include a cyclopropyl group, a 1-methylcyclopropyl group, a 2-methylcyclopropyl group, a 1-ethylcyclopropyl group, a 2-ethylcyclopropyl group, a 2,2-dimethylpropyl group, a 1,2,2-trimethylpropyl group, a cyclobutyl group, a 1-methylcyclobutyl group, a 2-methylcyclobutyl group, a 3-methylcyclobutyl group, a cyclopentyl group, a 1-methylcyclopentyl group, a 2-methylcyclopentyl group, a 3-methylcyclopentyl group, a cyclohexyl group, a 1-methylcyclohexyl group, a 2-methylcyclohexyl group, a 3-methylcyclohexyl group, a 4-methylcyclohexyl group, a 5-methylcyclohexyl group, a 6-methylcyclohexyl group, a 3,3-dimethylcyclohexyl group, a 4,4-dimethylcyclohexyl group, a cycloheptyl group, a 1-methylcycloheptyl group, a 2-methylcycloheptyl group, a 3-methylcycloheptyl group, a 4-methylcycloheptyl group, a cyclooctyl group and the like.

Examples of the C1-C6 alkoxy group represented by X or the C1-C6 alkoxy group as a substituent include a methoxy group, an ethoxy group, an n-propoxy group, an isopropoxy group, an n-butoxy group, an isobutoxy group, a sec-butoxy group, a tert-butoxy group and the like. The number of the C1-C6 alkoxy group(s) as a substituent may be one, two or more, and when the number is two or more, the C1-C6 alkoxy groups may be the same or different from each other. The position of substitution with the C1-C6 alkoxy group may be any position.

In a preferable embodiment of the isoxazolin-5-one derivatives represented by formula (1) above, $R^1$ represents a C1-C6 fluoroalkyl group, and in a more preferable embodiment, $R^1$ represents a trifluoromethyl group.

In the isoxazolin-5-one derivatives represented by formula (1) above, a combination of $R^1$ to $R^3$, Q, X and n is not particularly limited, but for example, an embodiment is as follows.

In formula (1) above, $R^1$ represents a trifluoromethyl group,

$R^2$ represents a hydrogen atom, a C1-C6 alkylcarbonyl group, a benzoyl group (the group may be monosubstituted or polysubstituted with a halogen atom(s) or a C1-C6 alkyl group(s)), a C1-C6 alkoxycarbonyl group, a phenoxycarbonyl group (the group maybe monosubstituted or polysubstituted with a halogen atom(s) or a C1-C6 alkyl group(s)) or a C1-C6 haloalkylsulfonyl group,

$R^3$ represents a C1-C6 alkyl group,

Q represents a 2-methoxycyclopentyl group or a 2-methoxycyclohexyl group,

X represents a hydrogen atom, and

n represents an integer of 1 to 4.

Although representative examples of the isoxazolin-5-one derivatives represented by formula (1) are shown together in Table 1 below, the isoxazolin-5-one derivatives are not limited to these compounds. The compounds include compounds including optical isomers, an E-form and a Z-form. The compound numbers are referred to in the following paragraphs.

The symbols below in the tables stand for the corresponding groups as follows.

"H" stands for a hydrogen atom. "Me" stands for a methyl group. "Et" stands for an ethyl group. "n-Pr" stands for a normal propyl group. "i-Pr" stands for an isopropyl group. "c-Pr" stands for a cyclopropyl group. "n-Bu" stands for a normal butyl group. "s-Bu" stands for a sec-butyl group. "i-Bu" stands for an isobutyl group. "c-Bu" stands for a cyclobutyl group. "t-Bu" stands for a tert-butyl group. "n-Pen" stands for a normal pentyl group. "c-Pen" stands for a cyclopentyl group. "n-Hex" stands for a normal hexyl group. "c-Hex" stands for a cyclohexyl group. "c-Hep" stands for a cycloheptyl group. "c-Oct" stands for a cyclooctyl group. "Ph" stands for a phenyl group. "Bz" stands for a benzoyl group.

[Table 1-1]

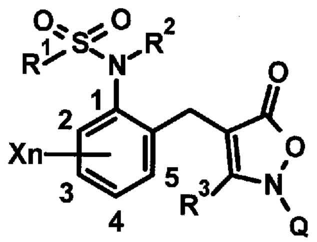

Table 1

| No. | $R^1$ | $R^2$ | $R^3$ | Q | Xn |
|---|---|---|---|---|---|
| **1** | $CH_2Cl$ | H | Me | 2-OMe(c-Pen) | H |
| **2** | $CH_2Cl$ | H | Me | 2-OMe(c-Hex) | H |
| **3** | $CCl_3$ | H | Me | 2-OMe(c-Pen) | H |
| **4** | $CCl_3$ | H | Me | 2-OMe(c-Hex) | H |
| **5** | $CH_2F$ | H | Me | 2-OMe(c-Pen) | H |
| **6** | $CH_2F$ | H | Me | 2-OMe(c-Hex) | H |
| **7** | $CF_2H$ | H | Me | 2-OMe(c-Pen) | H |
| **8** | $CF_2H$ | H | Me | 2-OMe(c-Hex) | H |
| **9** | $CF_3$ | H | H | 2-OMe(c-Pen) | H |

(continued)

| Table 1 | | | | | |
|---|---|---|---|---|---|
| **No.** | **$R^1$** | **$R^2$** | **$R^3$** | **Q** | **Xn** |
| **10** | $CF_3$ | H | H | 2-OMe(c-Hex) | H |
| **11** | $CF_3$ | H | F | 2-OMe(c-Pen) | H |
| **12** | $CF_3$ | H | F | 2-OMe(c-Hex) | H |
| **13** | $CF_3$ | H | Cl | 2-OMe(c-Pen) | H |
| **14** | $CF_3$ | H | Cl | 2-OMe(c-Hex) | H |
| **15** | $CF_3$ | H | Et | 2-OMe(c-Pen) | H |
| **16** | $CF_3$ | H | Et | 2-OMe(c-Hex) | H |
| **17** | $CF_3$ | H | n-Pr | 2-OMe(c-Pen) | H |
| **18** | $CF_3$ | H | n-Pr | 2-OMe(c-Hex) | H |
| **19** | $CF_3$ | H | i-Pr | 2-OMe(c-Pen) | H |
| **20** | $CF_3$ | H | i-Pr | 2-OMe(c-Hex) | H |
| **21** | $CF_3$ | H | t-Bu | 2-OMe(c-Pen) | H |
| **22** | $CF_3$ | H | t-Bu | 2-OMe(c-Hex) | H |
| **23** | $CF_3$ | H | $CF_2H$ | 2-OMe(c-Pen) | H |
| **24** | $CF_3$ | H | $CF_2H$ | 2-OMe(c-Hex) | H |
| **25** | $CF_3$ | H | $CF_3$ | 2-OMe(c-Pen) | H |
| **26** | $CF_3$ | H | $CF_3$ | 2-OMe(c-Hex) | H |
| **27** | $CF_3$ | H | c-Pr | 2-OMe(c-Pen) | H |
| **28** | $CF_3$ | H | c-Pr | 2-OMe(c-Hex) | H |
| **29** | $CF_3$ | H | Ph | 2-OMe(c-Pen) | H |
| **30** | $CF_3$ | H | Ph | 2-OMe(c-Hex) | H |
| **31** | $CF_3$ | H | 4-ClPh | 2-OMe(c-Pen) | H |
| **32** | $CF_3$ | H | 4-ClPh | 2-OMe(c-Hex) | H |
| **33** | $CF_3$ | H | 4-MePh | 2-OMe(c-Pen) | H |
| **34** | $CF_3$ | H | 4-MePh | 2-OMe(c-Hex) | H |
| **35** | $CF_3$ | H | $NH_2$ | 2-OMe(c-Pen) | H |

[Table 1-2]

| **No.** | **$R^1$** | **$R^2$** | **$R^3$** | **Q** | **Xn** |
|---|---|---|---|---|---|
| **36** | $CF_3$ | H | $NH_2$ | 2-OMe(c-Hex) | H |
| **37** | $CF_3$ | H | NHMe | 2-OMe(c-Pen) | H |
| **38** | $CF_3$ | H | NHMe | 2-OMe(c-Hex) | H |
| **39** | $CF_3$ | H | $NMe_2$ | 2-OMe(c-Pen) | H |
| **40** | $CF_3$ | H | $NMe_2$ | 2-OMe(c-Hex) | H |
| **41** | $CF_3$ | H | Me | 2-OMe(c-Pen) | 4-F |
| **42** | $CF_3$ | H | Me | 2-OMe(c-Hex) | 4-F |

(continued)

| No. | $R^1$ | $R^2$ | $R^3$ | Q | Xn |
|---|---|---|---|---|---|
| **43** | $CF_3$ | H | Me | 2-OMe(c-Pen) | 2-Cl |
| **44** | $CF_3$ | H | Me | 2-OMe(c-Hex) | 2-Cl |
| **45** | $CF_3$ | H | Me | 2-OMe(c-Pen) | 3-Cl |
| **46** | $CF_3$ | H | Me | 2-OMe(c-Hex) | 3-Cl |
| **47** | $CF_3$ | H | Me | 2-OMe(c-Pen) | 4-Cl |
| **48** | $CF_3$ | H | Me | 2-OMe(c-Hex) | 4-Cl |
| **49** | $CF_3$ | H | Me | 2-OMe(c-Pen) | 5-Cl |
| **50** | $CF_3$ | H | Me | 2-OMe(c-Hex) | 5-Cl |
| **51** | $CF_3$ | H | Me | 2-OMe(c-Pen) | 3,4-$Cl_2$ |
| **52** | $CF_3$ | H | Me | 2-OMe(c-Hex) | 3,4-$Cl_2$ |
| **53** | $CF_3$ | H | Me | 2-OMe(c-Pen) | 4-Me |
| **54** | $CF_3$ | H | Me | 2-OMe(c-Hex) | 4-Me |
| **55** | $CF_3$ | H | Me | 2-OMe(c-Pen) | 4-MeO |
| **56** | $CF_3$ | H | Me | 2-OMe(c-Hex) | 4-MeO |
| **57** | $CF_3$ | H | Me | 2-OMe(c-Pr) | H |
| **58** | $CF_3$ | H | Me | 2-OMe(c-Bu) | H |
| **59** | $CF_3$ | H | Me | 2-OMe(c-Pen) | H |
| **60** | $CF_3$ | H | Me | 3-OMe(c-Pen) | H |
| **61** | $CF_3$ | H | Me | 1-OMe(c-Hex) | H |
| **62** | $CF_3$ | H | Me | 2-OMe(c-Hex) | H |
| **63** | $CF_3$ | H | Me | 3-OMe(c-Hex) | H |
| **64** | $CF_3$ | H | Me | 4-OMe(c-Hex) | H |
| **65** | $CF_3$ | H | Me | 2-OMe-1-Me(c-Hex) | H |
| **66** | $CF_3$ | H | Me | 2-OMe-2-Me(c-Hex) | H |
| **67** | $CF_3$ | H | Me | 2-OMe-3-Me(c-Hex) | H |
| **68** | $CF_3$ | H | Me | 2-OMe-4-Me(c-Hex) | H |
| **69** | $CF_3$ | H | Me | 2-OMe-5-Me(c-Hex) | H |
| **70** | $CF_3$ | H | Me | 2-OMe-6-Mec-Hex) | H |
| **71** | $CF_3$ | H | Me | 2-OMe-3,3-$Me_2$(c-Hex) | H |
| **72** | $CF_3$ | H | Me | 2-OMe-4,4-$Me_2$(c-Hex) | H |
| **73** | $CF_3$ | H | Me | 2-OMe(c-Hep) | H |
| **74** | $CF_3$ | H | Me | 2-OMe(c-Oct) | H |
| **75** | $CF_3$ | Me | Me | 2-OMe(c-Pen) | H |

[Table 1-3]

| No. | $R^1$ | $R^2$ | $R^3$ | Q | Xn |
|---|---|---|---|---|---|
| **76** | $CF_3$ | Me | Me | 2-OMe(c-Hex) | H |

(continued)

| No. | $R^1$ | $R^2$ | $R^3$ | Q | Xn |
|---|---|---|---|---|---|
| **77** | $CF_3$ | $CH_2CF_3$ | Me | 2-OMe(c-Pen) | H |
| **78** | $CF_3$ | $CH_2CF_3$ | Me | 2-OMe(c-Hex) | H |
| **79** | $CF_3$ | $CH_2CH=CH_2$ | Me | 2-OMe(c-Pen) | H |
| **80** | $CF_3$ | $CH_2CH=CH_2$ | Me | 2-OMe(c-Hex) | H |
| **81** | $CF_3$ | $CH_2C\equiv CH$ | Me | 2-OMe(c-Pen) | H |
| **82** | $CF_3$ | $CH_2C\equiv CH$ | Me | 2-OMe(c-Hex) | H |
| **83** | $CF_3$ | $CH_2OMe$ | Me | 2-OMe(c-Pen) | H |
| **84** | $CF_3$ | $CH_2OMe$ | Me | 2-OMe(c-Hex) | H |
| **85** | $CF_3$ | $CH_2OCH_2CF_3$ | Me | 2-OMe(c-Pen) | H |
| **86** | $CF_3$ | $CH_2OCH_2CF_3$ | Me | 2-OMe(c-Hex) | H |
| **87** | $CF_3$ | $CH_2OCH_2CH_2OMe$ | Me | 2-OMe(c-Pen) | H |
| **88** | $CF_3$ | $CH_2OCH_2CH_2OMe$ | Me | 2-OMe(c-Hex) | H |
| **89** | $CF_3$ | $CH_2SMe$ | Me | 2-OMe(c-Pen) | H |
| **90** | $CF_3$ | $CH_2SMe$ | Me | 2-OMe(c-Hex) | H |
| **19** | $CF_3$ | | | | |
| **92** | $CF_3$ | $CH_2COMe$ | Me | 2-OMe(c-Hex) | H |
| **93** | $CF_3$ | $CH_2Ph$ | Me | 2-OMe(c-Pen) | H |
| **94** | $CF_3$ | $CH_2Ph$ | Me | 2-OMe(c-Hex) | H |
| **95** | $CF_3$ | $CH_2$(4-ClPh) | Me | 2-OMe(c-Pen) | H |
| **96** | $CF_3$ | $CH_2$(4-ClPh) | Me | 2-OMe(c-Hex) | H |
| **97** | $CF_3$ | $CH_2$(4-MePh) | Me | 2-OMe(c-Pen) | H |
| **98** | $CF_3$ | $CH_2$(4-MePh) | Me | 2-OMe(c-Hex) | H |
| **99** | $CF_3$ | $CH_2$(4-MeOPh) | Me | 2-OMe(c-Pen) | H |
| **100** | $CF_3$ | $CH_2$(4-MeOPh) | Me | 2-OMe(c-Hex) | H |
| **101** | $CF_3$ | $CH_2CH_2OPh$ | Me | 2-OMe(c-Pen) | H |
| **102** | $CF_3$ | $CH_2CH_2OPh$ | Me | 2-OMe(c-Hex) | H |
| **103** | $CF_3$ | $CH_2OCH_2Ph$ | Me | 2-OMe(c-Pen) | H |
| **104** | $CF_3$ | $CH_2OCH_2Ph$ | Me | 2-OMe(c-Hex) | H |
| **105** | $CF_3$ | $CH_2COPh$ | Me | 2-OMe(c-Pen) | H |
| **106** | $CF_3$ | $CH_2COPh$ | Me | 2-OMe(c-Hex) | H |
| **107** | $CF_3$ | COMe | Me | 2-OMe(c-Pen) | H |
| **108** | $CF_3$ | COMe | Me | 2-OMe(c-Hex) | H |
| **109** | $CF_3$ | COEt | Me | 2-OMe(c-Pen) | H |
| **110** | $CF_3$ | COEt | Me | 2-OMe(c-Hex) | H |
| **111** | $CF_3$ | COn-Pr | Me | 2-OMe(c-Pen) | H |
| **112** | $CF_3$ | COn-Pr | Me | 2-OMe(c-Hex) | H |
| **113** | $CF_3$ | COi-Pr | Me | 2-OMe(c-Pen) | H |
| **114** | $CF_3$ | COi-Pr | Me | 2-OMe(c-Hex) | H |

(continued)

| No. | $R^1$ | $R^2$ | $R^3$ | Q | Xn |
|---|---|---|---|---|---|
| **115** | $CF_3$ | COn-Bu | Me | 2-OMe(c-Pen) | H |

[Table 1-4]

| No. | $R^1$ | $R^2$ | $R^3$ | Q | Xn |
|---|---|---|---|---|---|
| **116** | $CF_3$ | COn-Bu | Me | 2-OMe(c-Hex) | H |
| **117** | $CF_3$ | COs-Bu | Me | 2-OMe(c-Pen) | H |
| **118** | $CF_3$ | COs-Bu | Me | 2-OMe(c-Hex) | H |
| **119** | $CF_3$ | COi-Bu | Me | 2-OMe(c-Pen) | H |
| **120** | $CF_3$ | COi-Bu | Me | 2-OMe(c-Hex) | H |
| **121** | $CF_3$ | COt-Bu | Me | 2-OMe(c-Pen) | H |
| **122** | $CF_3$ | COt-Bu | Me | 2-OMe(c-Hex) | H |
| **123** | $CF_3$ | $COCF_3$ | Me | 2-OMe(c-Pen) | H |
| **124** | $CF_3$ | $COCF_3$ | Me | 2-OMe(c-Hex) | H |
| **125** | $CF_3$ | $COCH=CH_2$ | Me | 2-OMe(c-Pen) | H |
| **126** | $CF_3$ | $COCH=CH_2$ | Me | 2-OMe(c-Hex) | H |
| **127** | $CF_3$ | $COC\equiv CH$ | Me | 2-OMe(c-Pen) | H |
| **128** | $CF_3$ | $COC\equiv CH$ | Me | 2-OMe(c-Hex) | H |
| **129** | $CF_3$ | COc-Pr | Me | 2-OMe(c-Pen) | H |
| **130** | $CF_3$ | COc-Pr | Me | 2-OMe(c-Hex) | H |
| **131** | $CF_3$ | COc-Hex | Me | 2-OMe(c-Pen) | H |
| **132** | $CF_3$ | COc-Hex | Me | 2-OMe(c-Hex) | H |
| **133** | $CF_3$ | $COCH_2$c-Pr | Me | 2-OMe(c-Pen) | H |
| **134** | $CF_3$ | $COCH_2$c-Pr | Me | 2-OMe(c-Hex) | H |
| **135** | $CF_3$ | $COCH_2OMe$ | Me | 2-OMe(c-Pen) | H |
| **136** | $CF_3$ | $COCH_2OMe$ | Me | 2-OMe(c-Hex) | H |
| **137** | $CF_3$ | $COCH_2OCH_2CF_3$ | Me | 2-OMe(c-Pen) | H |
| **138** | $CF_3$ | $COCH_2OCH_2CF_3$ | Me | 2-OMe(c-Hex) | H |
| **139** | $CF_3$ | $COCH_2OCH_2CH_2OMe$ | Me | 2-OMe(c-Pen) | H |
| **140** | $CF_3$ | $COCH_2OCH_2CH_2OMe$ | Me | 2-OMe(c-Hex) | H |
| **141** | $CF_3$ | $COCH_2SMe$ | Me | 2-OMe(c-Pen) | H |
| **142** | $CF_3$ | $COCH_2SMe$ | Me | 2-OMe(c-Hex) | H |
| **143** | $CF_3$ | $COCH_2SCH_2CF_3$ | Me | 2-OMe(c-Pen) | H |
| **144** | $CF_3$ | $COCH_2SCH_2CF_3$ | Me | 2-OMe(c-Hex) | H |
| **145** | $CF_3$ | Bz | Me | 2-OMec-Pen) | H |
| **146** | $CF_3$ | Bz | Me | 2-OMe(c-Hex) | H |
| **147** | $CF_3$ | 4-ClBz | Me | 2-OMe(c-Pen) | H |
| **148** | $CF_3$ | 4-ClBz | Me | 2-OMe(c-Hex) | H |

(continued)

| No. | $R^1$ | $R^2$ | $R^3$ | Q | Xn |
|---|---|---|---|---|---|
| **149** | $CF_3$ | 4-MeBz | Me | 2-OMe(c-Pen) | H |
| **150** | $CF_3$ | 4-MeBz | Me | 2-OMe(c-Hex) | H |
| **151** | $CF_3$ | $COCH_2Ph$ | Me | 2-OMe(c-Pen) | H |
| **152** | $CF_3$ | $COCH_2Ph$ | Me | 2-OMe(c-Hex) | H |
| **153** | $CF_3$ | $COCH_2$(4-ClPh) | Me | 2-OMe(c-Pen) | H |
| **154** | $CF_3$ | $COCH_2$(4-ClPh) | Me | 2-OMe(c-Hex) | H |
| **155** | $CF_3$ | $COCH_2$(4-MePh) | Me | 2-OMe(c-Pen) | H |

[Table 1-5]

| No. | $R^1$ | $R^2$ | $R^3$ | Q | Xn |
|---|---|---|---|---|---|
| **156** | $CF_3$ | $COCH_2$(4-MePh) | Me | 2-OMe(c-Hex) | H |
| **157** | $CF_3$ | CO(2-tetrahydrofuryl) | Me | 2-OMe(c-Pen) | H |
| **158** | $CF_3$ | CO(2-tetrahydrofuryl) | Me | 2-OMe(c-Hex) | H |
| **159** | $CF_3$ | CO(2-pyridyl) | Me | 2-OMe(c-Pen) | H |
| **160** | $CF_3$ | CO(2-pyridyl) | Me | 2-OMe(c-Hex) | H |
| **161** | $CF_3$ | CO(3-pyridyl) | Me | 2-OMec-Pen | H |
| **162** | $CF_3$ | CO(3-pyridyl) | Me | 2-OMe(c-Hex) | H |
| **163** | $CF_3$ | CO(4-pyridyl) | Me | 2-OMe(c-Pen) | H |
| **164** | $CF_3$ | CO(4-pyridyl) | Me | 2-OMe(c-Hex) | H |
| **165** | $CF_3$ | CO(2-thienyl) | Me | 2-OMe(c-Pen) | H |
| **166** | $CF_3$ | CO(2-thienyl) | Me | 2-OMe(c-Hex) | H |
| **167** | $CF_3$ | CO(3-thienyl) | Me | 2-OMe(c-Pen) | H |
| **168** | $CF_3$ | CO(3-thienyl) | Me | 2-OMe(c-Hex) | H |
| **169** | $CF_3$ | CO(2-tetrahydrofurfuryl) | Me | 2-OMe(c-Pen) | H |
| **170** | $CF_3$ | CO(2-tetrahydrofurfuryl) | Me | 2-OMe(c-Hex) | H |
| **171** | $CF_3$ | $COCH_2$(2-pyridyl) | Me | 2-OMe(c-Pen) | H |
| **172** | $CF_3$ | $COCH_2$(2-pyridyl) | Me | 2-OMe(c-Hex) | H |
| **173** | $CF_3$ | $COCH_2$(2-thienyl) | Me | 2-OMe(c-Pen) | H |
| **174** | $CF_3$ | $COCH_2$(2-thienyl) | Me | 2-OMe(c-Hex) | H |
| **175** | $CF_3$ | $CO_2Me$ | Me | 2-OMe(c-Bu) | H |
| **176** | $CF_3$ | $CO_2Me$ | Me | 2-OMe(c-Pen) | H |
| **177** | $CF_3$ | $CO_2Me$ | Me | 3-OMe(c-Pen) | H |
| **178** | $CF_3$ | $CO_2Me$ | Me | 2-OMe(c-Hex) | H |
| **179** | $CF_3$ | $CO_2Me$ | Me | 3-OMe(c-Hex) | H |
| **180** | $CF_3$ | $CO_2Me$ | Me | 4-OMe(c-Hex) | H |
| **181** | $CF_3$ | $CO_2Me$ | Me | 2-OMe-1-Me(c-Hex) | H |
| **182** | $CF_3$ | $CO_2Me$ | Me | 2-OMe-2-Me(c-Hex) | H |

(continued)

| No. | $R^1$ | $R^2$ | $R^3$ | Q | Xn |
|---|---|---|---|---|---|
| **183** | $CF_3$ | $CO_2Me$ | Me | 2-OMe-3-Me(c-Hex) | H |
| **184** | $CF_3$ | $CO_2Me$ | Me | 2-OMe-4-Me(c-Hex) | H |
| **185** | $CF_3$ | $CO_2Me$ | Me | 2-OMe(c-Hep) | H |
| **186** | $CF_3$ | $CO_2Me$ | Me | 2-OMe(c-Oct) | H |
| **187** | $CF_3$ | $CO_2Et$ | Me | 2-OMe(c-Bu) | H |
| **188** | $CF_3$ | $CO_2Et$ | Me | 2-OMe(c-Pen) | H |
| **189** | $CF_3$ | $CO_2Et$ | Me | 3-OMe(c-Pen) | H |
| **190** | $CF_3$ | $CO_2Et$ | Me | 2-OMe(c-Hex) | H |
| **191** | $CF_3$ | $CO_2Et$ | Me | 3-OMe(c-Hex) | H |
| **192** | $CF_3$ | $CO_2Et$ | Me | 4-OMe(c-Hex) | H |
| **193** | $CF_3$ | $CO_2Et$ | Me | 2-OMe-1-Me(c-Hex) | H |
| **194** | $CF_3$ | $CO_2Et$ | Me | 2-OMe-2-Me(c-Hex) | H |
| **195** | $CF_3$ | $CO_2Et$ | Me | 2-OMe-3-Me(c-Hex) | H |

[Table 1-6]

| No. | $R^1$ | $R^2$ | $R^3$ | Q | Xn |
|---|---|---|---|---|---|
| **196** | $CF_3$ | $CO_2Et$ | Me | 2-OMe-4-Me(c-Hex) | H |
| **197** | $CF_3$ | $CO_2Et$ | Me | 2-OMe(c-Hep) | H |
| **198** | $CF_3$ | $CO_2Et$ | Me | 2-OMe(c-Oct) | H |
| **199** | $CF_3$ | $CO_2n$-Pr | Me | 2-OMe(c-Bu) | H |
| **200** | $CF_3$ | $CO_2n$-Pr | Me | 2-OMe(c-Pen) | H |
| **201** | $CF_3$ | $CO_2n$-Pr | Me | 3-OMe(c-Pen) | H |
| **202** | $CF_3$ | $CO_2n$-Pr | Me | 2-OMe(c-Hex) | H |
| **203** | $CF_3$ | $CO_2n$-Pr | Me | 3-OMe(c-Hex) | H |
| **204** | $CF_3$ | $CO_2n$-Pr | Me | 4-OMe(c-Hex) | H |
| **205** | $CF_3$ | $CO_2n$-Pr | Me | 2-OMe-1-Me(c-Hex) | H |
| **206** | $CF_3$ | $CO_2n$-Pr | Me | 2-OMe-2-Me(c-Hex) | H |
| **207** | $CF_3$ | $CO_2n$-Pr | Me | 2-OMe-3-Me(c-Hex) | H |
| **208** | $CF_3$ | $CO_2n$-Pr | Me | 2-OMe-4-Me(c-Hex) | H |
| **209** | $CF_3$ | $CO_2n$-Pr | Me | 2-OMe(c-He) | H |
| **210** | $CF_3$ | $CO_2n$-Pr | Me | 2-OMe(c-Oct) | H |
| **211** | $CF_3$ | $CO_2i$-Pr | Me | 2-OMe(c-Bu) | H |
| **212** | $CF_3$ | $CO_2i$-Pr | Me | 2-OMe(c-Pen) | H |
| **213** | $CF_3$ | $CO_2i$-Pr | Me | 3-OMe(c-Pen) | H |
| **214** | $CF_3$ | $CO_2i$-Pr | Me | 2-OMe(c-Hex) | H |
| **215** | $CF_3$ | $CO_2i$-Pr | Me | 3-OMe(c-Hex) | H |
| **216** | $CF_3$ | $CO_2i$-Pr | Me | 4-OMe(c-Hex) | H |

(continued)

| No. | $R^1$ | $R^2$ | $R^3$ | Q | Xn |
|---|---|---|---|---|---|
| **217** | $CF_3$ | $CO_2$i-Pr | Me | 2-OMe-1-Me(c-Hex) | H |
| **218** | $CF_3$ | $CO_2$i-Pr | Me | 2-OMe-2-Me(c-Hex) | H |
| **219** | $CF_3$ | $CO_2$i-Pr | Me | 2-OMe-3-Me(c-Hex) | H |
| **220** | $CF_3$ | $CO_2$i-Pr | Me | 2-OMe-4-Me(c-Hex) | H |
| **221** | $CF_3$ | $CO_2$i-Pr | Me | 2-OMe(c-Hep) | H |
| **222** | $CF_3$ | $CO_2$i-Pr | Me | 2-OMe(c-Oct) | H |
| **223** | $CF_3$ | $CO_2$n-Bu | Me | 2-OMe(c-Bu) | H |
| **224** | $CF_3$ | $CO_2$n-Bu | Me | 2-OMe(c-Pen) | H |
| **225** | $CF_3$ | $CO_2$n-Bu | Me | 3-OMe(c-Pen) | H |
| **226** | $CF_3$ | $CO_2$n-Bu | Me | 2-OMe(c-Hex) | H |
| **227** | $CF_3$ | $CO_2$n-Bu | Me | 3-OMe(c-Hex) | H |
| **228** | $CF_3$ | $CO_2$n-Bu | Me | 4-OMe(c-Hex) | H |
| **229** | $CF_3$ | $CO_2$n-Bu | Me | 2-OMe-1-Me(c-Hex) | H |
| **230** | $CF_3$ | $CO_2$n-Bu | Me | 2-OMe-2-Me(c-Hex) | H |
| **231** | $CF_3$ | $CO_2$n-Bu | Me | 2-OMe-3-Me(c-Hex) | H |
| **232** | $CF_3$ | $CO_2$n-Bu | Me | 2-OMe-4-Me(c-Hex) | H |
| **233** | $CF_3$ | $CO_2$n-Bu | Me | 2-OMe(c-Hep) | H |
| **234** | $CF_3$ | $CO_2$n-Bu | Me | 2-OMe(c-Oct) | H |
| **235** | $CF_3$ | $CO_2$s-Bu | Me | 2-OMe(c-Bu) | H |

[Table 1-7]

| No. | $R^1$ | $R^2$ | $R^3$ | Q | Xn |
|---|---|---|---|---|---|
| **236** | $CF_3$ | $CO_2$s-Bu | Me | 2-OMe(c-Pen) | H |
| **237** | $CF_3$ | $CO_2$s-Bu | Me | 3-OMe(c-Pen) | H |
| **238** | $CF_3$ | $CO_2$s-Bu | Me | 2-OMe(c-Hex) | H |
| **239** | $CF_3$ | $CO_2$s-Bu | Me | 3-OMe(c-Hex) | H |
| **240** | $CF_3$ | $CO_2$s-Bu | Me | 4-OMe(c-Hex) | H |
| **241** | $CF_3$ | $CO_2$s-Bu | Me | 2-OMe-1-Me(c-Hex) | H |
| **242** | $CF_3$ | $CO_2$s-Bu | Me | 2-OMe-2-Me(c-Hex) | H |
| **243** | $CF_3$ | $CO_2$s-Bu | Me | 2-OMe-3-Me(c-Hex) | H |
| **244** | $CF_3$ | $CO_2$s-Bu | Me | 2-OMe-4-Me(c-Hex) | H |
| **245** | $CF_3$ | $CO_2$s-Bu | Me | 2-OMe(c-Hep) | H |
| **246** | $CF_3$ | $CO_2$s-Bu | Me | 2-OMe(c-Oct) | H |
| **247** | $CF_3$ | $CO_2$i-Bu | Me | 2-OMe(c-Bu) | H |
| **248** | $CF_3$ | $CO_2$i-Bu | Me | 2-OMe(c-Pen) | H |
| **249** | $CF_3$ | $CO_2$i-Bu | Me | 3-OMe(c-Pen) | H |
| **250** | $CF_3$ | $CO_2$i-Bu | Me | 2-OMe(c-Hex) | H |

(continued)

| No. | $R^1$ | $R^2$ | $R^3$ | Q | Xn |
|---|---|---|---|---|---|
| **251** | $CF_3$ | $CO_2$i-Bu | Me | 3-OMe(c-Hex) | H |
| **252** | $CF_3$ | $CO_2$i-Bu | Me | 4-OMe(c-Hex) | H |
| **253** | $CF_3$ | $CO_2$i-Bu | Me | 2-OMe-1-Me(c-Hex) | H |
| **254** | $CF_3$ | $CO_2$i-Bu | Me | 2-OMe-2-Me(c-Hex) | H |
| **255** | $CF_3$ | $CO_2$i-Bu | Me | 2-OMe-3-Me(c-Hex) | H |
| **256** | $CF_3$ | $CO_2$i-Bu | Me | 2-OMe-4-Me(c-Hex) | H |
| **257** | $CF_3$ | $CO_2$i-Bu | Me | 2-OMe(c-Hep) | H |
| **258** | $CF_3$ | $CO_2$i-Bu | Me | 2-OMe(c-Oct) | H |
| **259** | $CF_3$ | $CO_2$t-Bu | Me | 2-OMe(c-Pen) | H |
| **260** | $CF_3$ | $CO_2$t-Bu | Me | 2-OMe(c-Hex) | H |
| **261** | $CF_3$ | $CO_2$n-Pen | Me | 2-OMe(c-Pen) | H |
| **262** | $CF_3$ | $CO_2$n-Pen | Me | 2-OMe(c-Hex) | H |
| **263** | $CF_3$ | $CO_2$n-Hex | Me | 2-OMe(c-Pen) | H |
| **264** | $CF_3$ | $CO_2$n-Hex | Me | 2-OMe(c-Hex) | H |
| **265** | $CF_3$ | $CO_2CH_2CF_3$ | Me | 2-OMe(c-Pen) | H |
| **266** | $CF_3$ | $CO_2CH_2CF_3$ | Me | 2-OMe(c-Hex) | H |
| **267** | $CF_3$ | $CO_2CH_2CH{=}CH_2$ | Me | 2-OMe(c-Pen) | H |
| **268** | $CF_3$ | $CO_2CH_2CH{=}CH_2$ | Me | 2-OMe(c-Hex) | H |
| **269** | $CF_3$ | $CO_2CH_2C{\equiv}CH$ | Me | 2-OMe(c-Pen) | H |
| **270** | $CF_3$ | $CO_2CH_2C{\equiv}CH$ | Me | 2-OMe(c-Hex) | H |
| **271** | $CF_3$ | $CO_2$c-Pr | Me | 2-OMe(c-Pen) | H |
| **272** | $CF_3$ | $CO_2$c-Pr | Me | 2-OMe(c-Hex) | H |
| **273** | $CF_3$ | $CO_2CH_2$c-Pr | Me | 2-OMe(c-Pen) | H |
| **274** | $CF_3$ | $CO_2CH_2$c-Pr | Me | 2-OMe(c-Hex) | H |
| **275** | $CF_3$ | $CO_2CH_2CH_2OMe$ | Me | 2-OMe(c-Pen) | H |

[Table 1-8]

| No. | $R^1$ | $R^2$ | $R^3$ | Q | Xn |
|---|---|---|---|---|---|
| **276** | $CF_3$ | $CO_2CH_2CH_2OMe$ | Me | 2-OMe(c-Hex) | H |
| **277** | $CF_3$ | $CO_2CH_2CH_2OCH_2CF_3$ | Me | 2-OMe(c-Pen) | H |
| **278** | $CF_3$ | $CO_2CH_2CH_2OCH_2CF_3$ | Me | 2-OMe(c-Hex) | H |
| **279** | $CF_3$ | $CO_2CH_2CH_2OCH_2CH_2OMe$ | Me | 2-OMe(c-Pen) | H |
| **280** | $CF_3$ | $CO_2CH_2CH_2OCH_2CH_2OMe$ | Me | 2-OMe(c-Hex) | H |
| **281** | $CF_3$ | $CO_2CH_2CH_2SMe$ | Me | 2-OMe(c-Pen) | H |
| **282** | $CF_3$ | $CO_2CH_2CH_2SMe$ | Me | 2-OMe(c-Hex) | H |
| **283** | $CF_3$ | $CO_2CH_2CH_2SCH_2CF_3$ | Me | 2-OMe(c-Pen) | H |
| **284** | $CF_3$ | $CO_2CH_2CH_2SCH_2CF_3$ | Me | 2-OMe(c-Hex) | H |

(continued)

| No. | $R^1$ | $R^2$ | $R^3$ | Q | Xn |
|---|---|---|---|---|---|
| **285** | $CF_3$ | $CO_2Ph$ | Me | 2-OMe(c-Bu) | H |
| **286** | $CF_3$ | $CO_2Ph$ | Me | 2-OMe(c-Pen) | H |
| **287** | $CF_3$ | $CO_2Ph$ | Me | 3-OMe(c-Pen) | H |
| **288 (TLC top)** | $CF_3$ | $CO_2Ph$ | Me | 2-OMe(c-Hex) | H |
| **289 (TLC bottom)** | $CF_3$ | $CO_2Ph$ | Me | 2-OMe(c-Hex) | H |
| **290** | $CF_3$ | $CO_2Ph$ | Me | 3-OMe(c-Hex) | H |
| **291** | $CF_3$ | $CO_2Ph$ | Me | 4-OMe(c-Hex) | H |
| **292** | $CF_3$ | $CO_2Ph$ | Me | 2-OMe-1-Me(c-Hex) | H |
| **293** | $CF_3$ | $CO_2Ph$ | Me | 2-OMe-2-Me(c-Hex) | H |
| **294** | $CF_3$ | $CO_2Ph$ | Me | 2-OMe-3-Me(c-Hex) | H |
| **295** | $CF_3$ | $CO_2Ph$ | Me | 2-OMe-4-Me(c-Hex) | H |
| **296** | $CF_3$ | $CO_2Ph$ | Me | 2-OMe(c-Hep) | H |
| **297** | $CF_3$ | $CO_2Ph$ | Me | 2-OMe(c-Oct) | H |
| **298** | $CF_3$ | $CO_2$(4-ClPh) | Me | 2-OMe(c-Pen) | H |
| **299** | $CF_3$ | $CO_2$(4-ClPh) | Me | 2-OMe(c-Hex) | H |
| **300** | $CF_3$ | $CO_2$(4-MePh) | Me | 2-OMe(c-Pen) | H |
| **301** | $CF_3$ | $CO_2$(4-MePh) | Me | 2-OMe(c-Hex) | H |
| **302** | $CF_3$ | $CO_2CH_2Ph$ | Me | 2-OMe(c-Pen) | H |
| **303** | $CF_3$ | $CO_2CH_2Ph$ | Me | 2-OMe(c-Hex) | H |
| **304** | $CF_3$ | $CO_2CH_2$(4-ClPh) | Me | 2-OMe(c-Pen) | H |
| **305** | $CF_3$ | $CO_2CH_2$(4-ClPh) | Me | 2-OMe(c-Hex) | H |
| **306** | $CF_3$ | $CO_2CH_2$(4-MePh) | Me | 2-OMe(c-Pen) | H |
| **307** | $CF_3$ | $CO_2CH_2$(4-MePh) | Me | 2-OMe(c-Hex) | H |
| **308** | $CF_3$ | $CO_2$(2-tetrahydrofuryl) | Me | 2-OMe(c-Pen) | H |
| **309** | $CF_3$ | $CO_2$(2-tetrahydrofuryl) | Me | 2-OMe(c-Hex) | H |
| **310** | $CF_3$ | $CO_2$(2-pyridyl) | Me | 2-OMe(c-Pen) | H |
| **311** | $CF_3$ | $CO_2$(2-pyridyl) | Me | 2-OMe(c-Hex) | H |
| **312** | $CF_3$ | $CO_2$(2-thienyl) | Me | 2-OMe(c-Pen) | H |
| **313** | $CF_3$ | $CO_2$(2-thienyl) | Me | 2-OMe(c-Hex) | H |
| **314** | $CF_3$ | $CO_2$(2-tetrahydrofurfuryl) | Me | 2-OMe(c-Pen) | H |
| **315** | $CF_3$ | $CO_2$(2-tetrahydrofurfuryl) | Me | 2-OMe(c-Hex) | H |

[Table 1-9]

| No. | $R^1$ | $R^2$ | $R^3$ | Q | Xn |
|---|---|---|---|---|---|
| **316** | $CF_3$ | $CO_2CH_2$(2-pyridyl) | Me | 2-OMe(c-Pen) | H |
| **317** | $CF_3$ | $CO_2CH_2$(2-pyridyl) | Me | 2-OMe(c-Hex) | H |
| **318** | $CF_3$ | $CO_2CH_2$(2-thienyl) | Me | 2-OMe(c-Pen) | H |

(continued)

| No. | $R^1$ | $R^2$ | $R^3$ | Q | Xn |
|---|---|---|---|---|---|
| **319** | $CF_3$ | $CO_2CH_2$(2-thienyl) | Me | 2-OMe(c-Hex) | H |
| **320** | $CF_3$ | CO(SMe) | Me | 2-OMe(c-Pen) | H |
| **321** | $CF_3$ | CO(SMe) | Me | 2-OMe(c-Hex) | H |
| **322** | $CF_3$ | $CO(SCH_2CF_3)$ | Me | 2-OMe(c-Pen) | H |
| **323** | $CF_3$ | $CO(SCH_2CF_3)$ | Me | 2-OMe(c-Hex) | H |
| **324** | $CF_3$ | CONHEt | Me | 2-OMe(c-Pen) | H |
| **325** | $CF_3$ | CONHEt | Me | 2-OMe(c-Hex) | H |
| **326** | $CF_3$ | $CONHCH_2CF_3$ | Me | 2-OMe(c-Pen) | H |
| **327** | $CF_3$ | $CONHCH_2CF_3$ | Me | 2-OMe(c-Hex) | H |
| **328** | $CF_3$ | $CONEt_2$ | Me | 2-OMe(c-Pen) | H |
| **329** | $CF_3$ | $CONEt_2$ | Me | 2-OMe(c-Hex) | H |
| **330** | $CF_3$ | $SO_2Me$ | Me | 2-OMe(c-Pen) | H |
| **331** | $CF_3$ | $SO_2Me$ | Me | 2-OMe(c-Hex) | H |
| **332** | $CF_3$ | $SO_2Et$ | Me | 2-OMe(c-Pen) | H |
| **333** | $CF_3$ | $SO_2Et$ | Me | 2-OMe(c-Hex) | H |
| **334** | $CF_3$ | $SO_2$n-Pr | Me | 2-OMe(c-Pen) | H |
| **335** | $CF_3$ | $SO_2$n-Pr | Me | 2-OMe(c-Hex) | H |
| **336** | $CF_3$ | $SO_2$i-Pr | Me | 2-OMe(c-Pen) | H |
| **337** | $CF_3$ | $SO_2$i-Pr | Me | 2-OMe(c-Hex) | H |
| **338** | $CF_3$ | $SO_2$n-Bu | Me | 2-OMe(c-Pen) | H |
| **339** | $CF_3$ | $SO_2$n-Bu | Me | 2-OMe(c-Hex) | H |
| **340** | $CF_3$ | $SO_2$i-Bu | Me | 2-OMe(c-Pen) | H |
| **341** | $CF_3$ | $SO_2$i-Bu | Me | 2-OMe(c-Hex) | H |
| **342** | $CF_3$ | $SO_2CH_2Cl$ | Me | 2-OMe(c-Pen) | H |
| **343** | $CF_3$ | $SO_2CH_2Cl$ | Me | 2-OMe(c-Hex) | H |
| **344** | $CF_3$ | $SO_2CCl_3$ | Me | 2-OMe(c-Pen) | H |
| **345** | $CF_3$ | $SO_2CCl_3$ | Me | 2-OMe(c-Hex) | H |
| **346** | $CF_3$ | $SO_2CHF_2$ | Me | 2-OMe(c-Pen) | H |
| **347** | $CF_3$ | $SO_2CHF_2$ | Me | 2-OMe(c-Hex) | H |
| **348** | $CF_3$ | $SO_2CF_3$ | Me | 2-OMe(c-Bu) | H |
| **349** | $CF_3$ | $SO_2CF_3$ | Me | 2-OMe(c-Pen) | H |
| **350** | $CF_3$ | $SO_2CF_3$ | Me | 3-OMe(c-Pen) | H |
| **351** | $CF_3$ | $SO_2CF_3$ | Me | 2-OMe(c-Hex) | H |
| **352** | $CF_3$ | $SO_2CF_3$ | Me | 3-OMe(c-Hex) | H |
| **353** | $CF_3$ | $SO_2CF_3$ | Me | 4-OMe(c-Hex) | H |
| **354** | $CF_3$ | $SO_2CF_3$ | Me | 2-OMe-1-Me(c-Hex) | H |
| **355** | $CF_3$ | $SO_2CF_3$ | Me | 2-OMe-2-Me(c-Hex) | H |

[Table 1-10]

| No. | $R^1$ | $R^2$ | $R^3$ | Q | Xn |
|---|---|---|---|---|---|
| **356** | $CF_3$ | $SO_2CF_3$ | Me | 2-OMe-3-Me(c-Hex) | H |
| **357** | $CF_3$ | $SO_2CF_3$ | Me | 2-OMe-4-Me(c-Hex) | H |
| **358** | $CF_3$ | $SO_2CF_3$ | Me | 2-OMe(c-Hep) | H |
| **359** | $CF_3$ | $SO_2CF_3$ | Me | 2-OMe(c-Oct) | H |
| **360** | $CF_3$ | $SO_2CH_2CF_3$ | Me | 2-OMe(c-Pen) | H |
| **361** | $CF_3$ | $SO_2CH_2CF_3$ | Me | 2-OMe(c-Hex) | H |
| **362** | $CF_3$ | $SO_2CH=CH_2$ | Me | 2-OMe(c-Pen) | H |
| **363** | $CF_3$ | $SO_2CH=CH_2$ | Me | 2-OMe(c-Hex) | H |
| **364** | $CF_3$ | $SO_2CH_2CH=CH_2$ | Me | 2-OMe(c-Pen) | H |
| **365** | $CF_3$ | $SO_2CH_2CH=CH_2$ | Me | 2-OMe(c-Hex) | H |
| **366** | $CF_3$ | $SO_2CH_2C{\equiv}CH$ | Me | 2-OMe(c-Pen) | H |
| **367** | $CF_3$ | $SO_2CH_2C{\equiv}CH$ | Me | 2-OMe(c-Hex) | H |
| **368** | $CF_3$ | $SO_2$c-Pr | Me | 2-OMe(c-Pen) | H |
| **369** | $CF_3$ | $SO_2$c-Pr | Me | 2-OMe(c-Hex) | H |
| **370** | $CF_3$ | $SO_2$c-Hex | Me | 2-OMe(c-Pen) | H |
| **371** | $CF_3$ | $SO_2$c-Hex | Me | 2-OMe(c-Hex) | H |
| **372** | $CF_3$ | $SO_2CH_2$c-Pr | Me | 2-OMe(c-Pen) | H |
| **373** | $CF_3$ | $SO_2CH_2$c-Pr | Me | 2-OMe(c-Hex) | H |
| **374** | $CF_3$ | $SO_2CH_2CH_2OMe$ | Me | 2-OMe(c-Pen) | H |
| **375** | $CF_3$ | $SO_2CH_2CH_2OMe$ | Me | 2-OMe(c-Hex) | H |
| **376** | $CF_3$ | $SO_2Ph$ | Me | 2-OMe(c-Pen) | H |
| **377** | $CF_3$ | $SO_2Ph$ | Me | 2-OMe(c-Hex) | H |
| **378** | $CF_3$ | $SO_2$(4-ClPh) | Me | 2-OMe(c-Pen) | H |
| **379** | $CF_3$ | $SO_2$(4-ClPh) | Me | 2-OMe(c-Hex) | H |
| **380** | $CF_3$ | $SO_2$(4-MePh) | Me | 2-OMe(c-Pen) | H |
| **381** | $CF_3$ | $SO_2$(4-MePh) | Me | 2-OMe(c-Hex) | H |
| **382** | $CF_3$ | $SO_2CH_2Ph$ | Me | 2-OMe(c-Pen) | H |
| **383** | $CF_3$ | $SO_2CH_2Ph$ | Me | 2-OMe(c-Hex) | H |
| **384** | $CF_3$ | $SO_2CH_2$(4-ClPh) | Me | 2-OMe(c-Pen) | H |
| **385** | $CF_3$ | $SO_2CH_2$(4-ClPh) | Me | 2-OMe(c-Hex) | H |
| **386** | $CF_3$ | $SO_2CH_2$(4-MePh) | Me | 2-OMe(c-Pen) | H |
| **387** | $CF_3$ | $SO_2CH_2$(4-MePh) | Me | 2-OMe(c-Hex) | H |
| **388** | $CF_3$ | $SO_2NHMe$ | Me | 2-OMe(c-Pen) | H |
| **389** | $CF_3$ | $SO_2NHMe$ | Me | 2-OMe(c-Hex) | H |
| **390** | $CF_3$ | $SO_2NMe_2$ | Me | 2-OMe(c-Pen) | H |
| **391** | $CF_3$ | $SO_2NMe_2$ | Me | 2-OMe(c-Hex) | H |

Next, although production methods of the isoxazolin-5-one derivatives represented by formula (1) of the invention (the compounds of the invention) are explained in detail, the production methods are not limited to these methods. In this regard, regarding the reaction devices, a reaction using a microwave synthesis device is also possible in addition to a reaction using a magnetic stirrer or a mechanical stirrer.

In the production methods below, the isoxazolin-5-one derivatives represented by formula (1a), (1b) or (1 c) correspond to the compounds of the invention.

[Production Method 1]

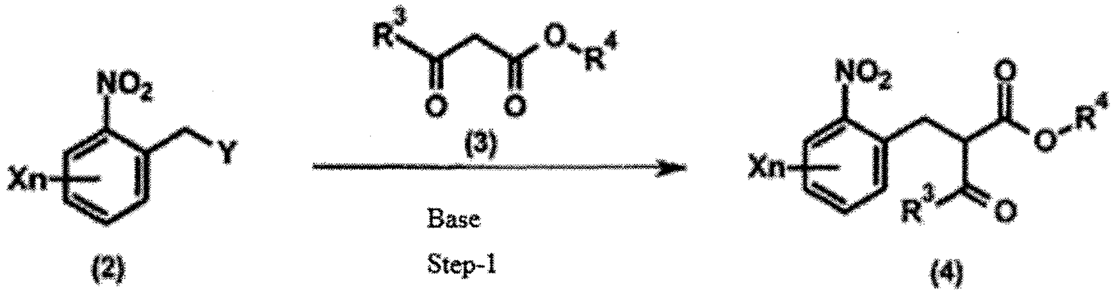


(2) (4)

($R^3$, X and n have the same meanings as those described above. Y represents a leaving group such as a halogen atom, a methanesulfonyloxy group, a trifluoromethanesulfonyloxy group or a toluenesulfonyloxy group. $R^4$ represents a C1-C6 alkyl group.)

The step-1 is a step of reacting a nitrobenzene derivative represented by formula (2) and a β-ketoester derivative represented by formula (3) in the presence of a base and thus producing a 2-(2-nitrobenzyl)-β-ketoester derivative (4). The nitrobenzene derivative represented by formula (2) and the β-ketoester derivative represented by formula (3) are sometimes known and can be obtained from Tokyo Chemical Industry Co., Ltd. or the like. Alternatively, the derivatives can also be easily produced from an available reagent according to a known method described in Courses in Experimental Chemistry, Organic Syntheses or the like.

It is necessary to conduct the reaction in the presence of a base, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. Of these bases, a metal base such as sodium methoxide and sodium ethoxide is preferable in view of the high yield. By conducting a reaction using the base in an amount of 0.1 to 5 equivalents based on the substrates, the target material can be obtained with a high yield. The reaction substrate (3) is generally used in an amount of 1 to 5 equivalents based on the substrate (2).

The reaction is preferably conducted in the presence of a solvent. A solvent which does not adversely affect the reaction can be used as the solvent used, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used. To promote the progress of the reaction, a phase-transfer catalyst such as a quaternary ammonium salt can also be added.

The reaction can be conducted at a temperature which is appropriately determined in the range of from -78°C to 200°C, although the temperature varies with the reaction conditions. After the reaction, the target material can be obtained by a general post-treatment operation, but the target material can also be purified by column chromatography, recrystallization or the like if necessary.

[Production Method 2]

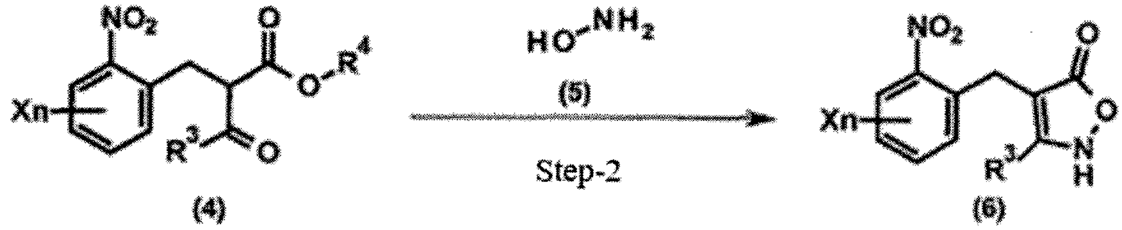


(4) (6)

($R^3$, X, n and $R^4$ have the same meanings as those described above.)

The step-2 is a step of reacting the 2-(2-nitrobenzyl)-β-ketoester derivative represented by formula (4) and hydroxylamine represented by formula (5) and thus producing an isoxazolin-5-one derivative (6). Hydroxylamine represented by formula (5) may be a quaternary salt such as a hydrochloride or a sulfate.

The reaction may be conducted in the presence of a base, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. By conducting the reaction using the base in an amount of 0.1 to 5 equivalents based on the substrates, the target material can be obtained with a high yield. The reaction substrate (5) is generally used in an amount of 1 to 5 equivalents based on the substrate (4).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

[Production Method 3]

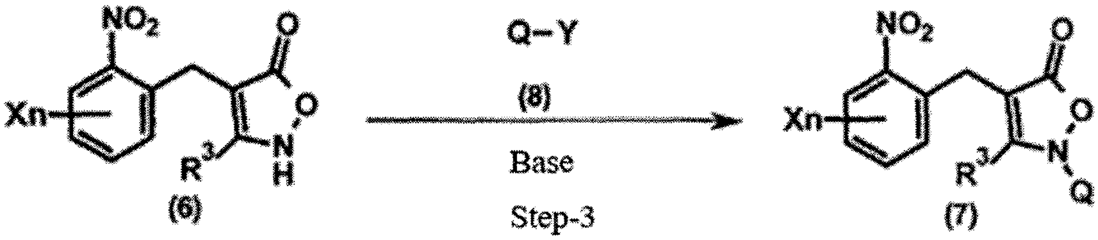


(6) (7)

($R^3$, Q, X, n and Y have the same meanings as those described above.)

The step-3 is a step of introducing Q to the nitrogen atom at the 2-position of the isoxazolin-5-one derivative represented by formula (6) and thus producing an isoxazolin-5-one derivative represented by formula (7).

It is necessary to conduct the reaction in the presence of a base, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used.

Of these bases, a metal base such as potassium carbonate and sodium hydride is preferable in view of the high yield. By conducting the reaction using the base in an amount of 0.1 to 5 equivalents based on the substrates, the target material can be obtained with a high yield. The reaction substrate (8) is generally used in an amount of 1 to 5 equivalents based on the substrate (6).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary. An O-substitution product may be generated in the reaction but can be easily separated and purified by column chromatography or the like.

[Production Method 4]

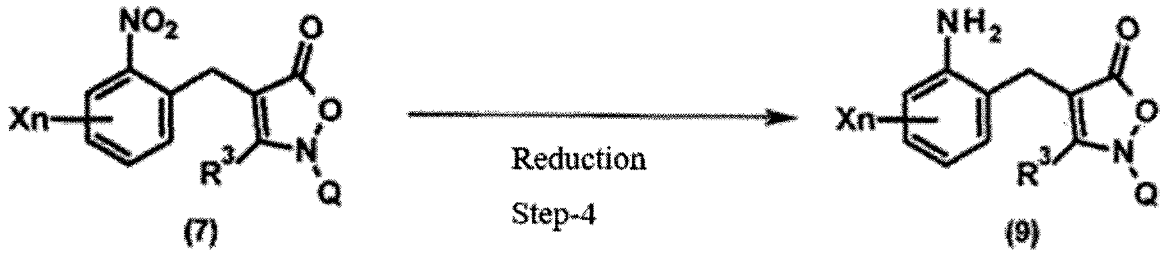


(7) (9)

($R^3$, Q, X and n have the same meanings as those described above.)

The step-4 is a step of reducing the nitro group of the isoxazolin-5-one derivative represented by formula (7) and thus producing an isoxazolin-5-one derivative (9) having an amino group.

The method for reducing the nitro group in the step can be a method using a reducing agent such as zinc powder, reduced iron, tin powder, stannous chloride and titanium chloride, a method using a hydrogen donor such as hydrazine in the presence of Raney nickel, catalytic hydrogenation reduction or catalytic hydrogen transfer reduction in the presence of a catalyst such as Raney nickel, palladium on carbon, palladium hydroxide and platinum oxide or the like.

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water, hydrochloric acid, acetic acid or a mixed solvent thereof can be used.

The reaction can be conducted at a temperature which is appropriately determined in the range of from 0°C to 200°C, although the temperature varies with the reaction conditions. After the reaction, the target material can be obtained by a general post-treatment operation, but the target material can also be purified by column chromatography, recrystallization or the like if necessary.

[Production Method 5]

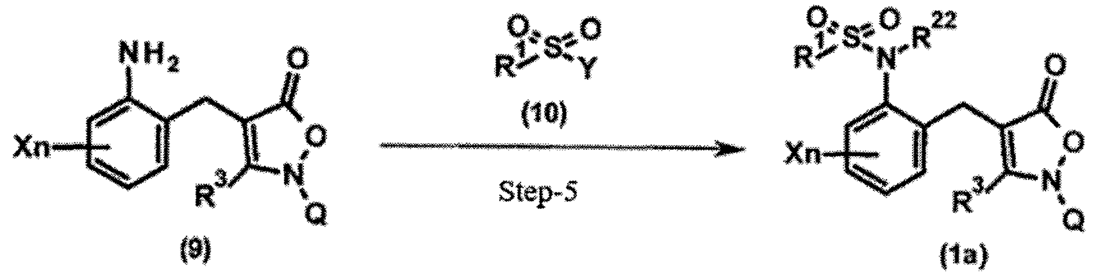


(9) (1a)

($R^1$, $R^3$, Q, X, n and Y have the same meanings as those described above. $R^{22}$ represents a hydrogen atom or $R^1SO_2$.)

The step-5 is a step of reacting the isoxazolin-5-one derivative having an amino group represented by formula (9) and a compound represented by formula (10) and thus producing an isoxazolin-5-one derivative (1a).

The reaction may be conducted in the presence of a base, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. Of these bases, an organic base such as triethylamine and diisopropylethylamine is preferable in view of the high yield. By conducting the reaction using the base in an amount of 0.1 to 5 equivalents based on the substrates, the target material can be obtained with a high yield. The reaction substrate (10) is generally used in an amount of 1 to 5 equivalents based on the substrate (9).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

[Production Method 6]

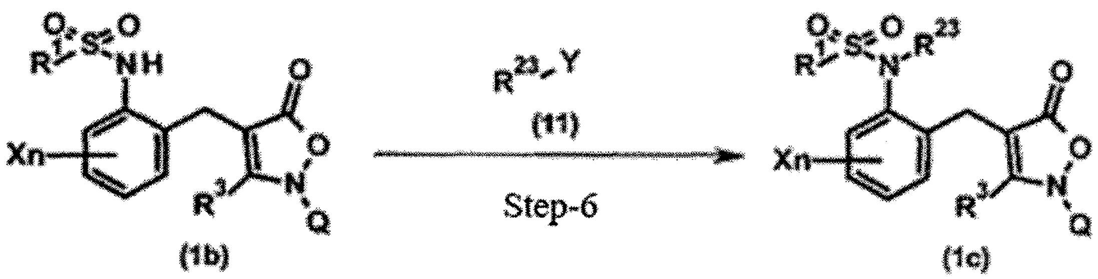


(1b) (1c)

($R^1$, $R^3$, Q, X, n and Y have the same meanings as those described above. $R^{23}$ represents any of the groups in $R^2$ except for a hydrogen atom.)

The step-6 is a step of reacting a compound represented by formula (11) with an isoxazolin-5-one derivative represented by formula (1b) and thus producing an isoxazolin-5-one derivative (1c).

It is necessary to conduct the reaction in the presence of a base depending on the kind of the compound (11), and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt

such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. By conducting the reaction using the base in an amount of 0.1 to 5 equivalents based on the substrates, the target material can be obtained with a high yield. The reaction substrate (11) is generally used in an amount of 1 to 5 equivalents based on the substrate (1b).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

[Production Method 7]

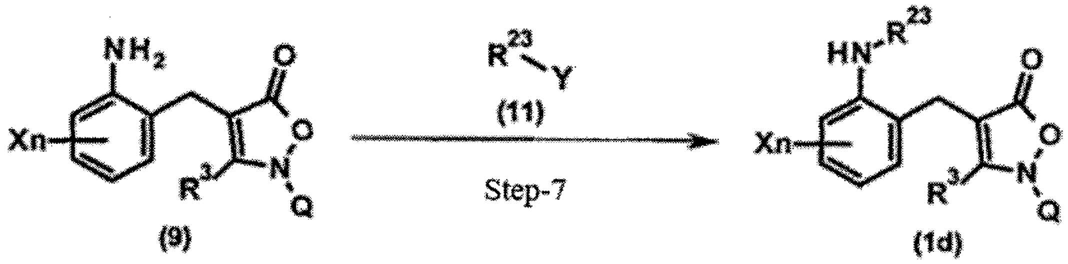


(9) (1d)

($R^3$, $R^{23}$, Q, X, n and Y have the same meanings as those described above.)

The step-7 is a step of reacting the compound represented by formula (11) with the isoxazolin-5-one derivative having an amino group represented by formula (9) and thus producing an isoxazolin-5-one derivative (1d).

It is necessary to conduct the reaction in the presence of a base depending on the kind of the compound (11), and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. By conducting the reaction using the base in an amount of 0.1 to 5 equivalents based on the substrates, the target material can be obtained with a high yield. The reaction substrate (11) is generally used in an amount of 1 to 5 equivalents based on the substrate (9).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column

chromatography, recrystallization or the like if necessary.

[Production Method 8]

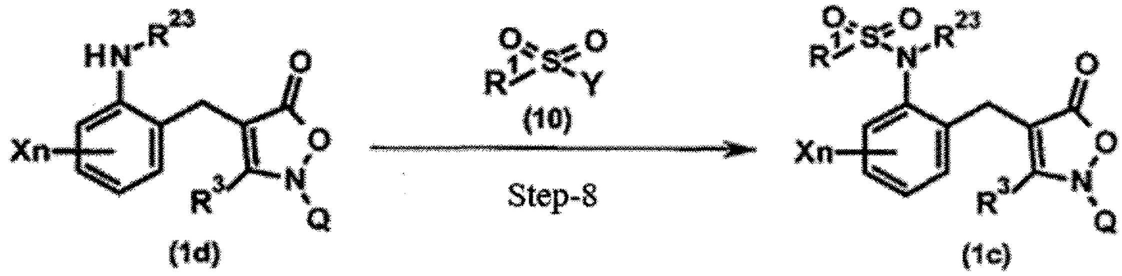


(1d) (1c)

($R^1$, $R^3$, $R^{23}$, Q, X, n and Y have the same meanings as those described above.)

The step-8 is a step of reacting the sulfonyl compound represented by formula (10) with the isoxazolin-5-one derivative represented by formula (1d) and thus producing the isoxazolin-5-one derivative (1c).

The reaction may be conducted in the presence of a base, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline and lutidine, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. By conducting the reaction using the base in an amount of 0.1 to 5 equivalents based on the substrates, the target material can be obtained with a high yield. The reaction substrate (10) is generally used in an amount of 1 to 5 equivalents based on the substrate (1d).

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

[Production Method 9]

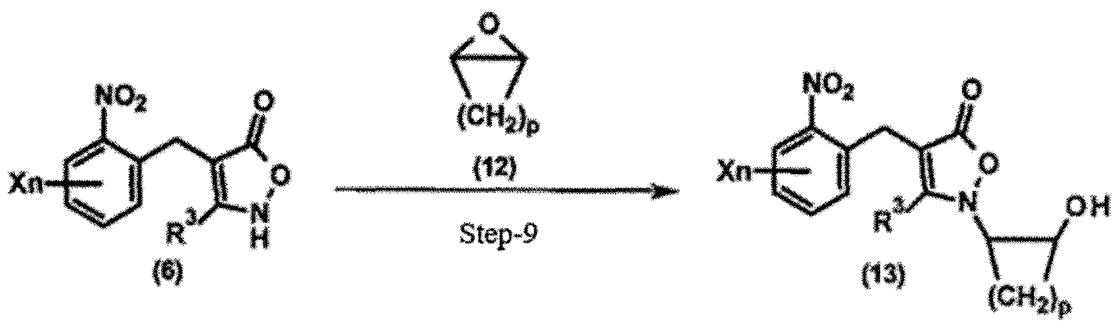


(6) (13)

($R^3$, X and n have the same meanings as those described above. p represents an integer of 1 to 6.)

The step-9 is a step of producing an isoxazolin-5-one derivative represented by formula (13) in which a hydroxycycloalkyl group has been introduced to the nitrogen atom at the 2-position of the isoxazolin-5-one derivative represented by formula (6) using a ring-opening reaction of an oxirane derivative (12).

The reaction may be conducted in the presence of a metal salt, and as the metal salt, a metal salt of a metal or a transition metal belonging to group 1, group 2, group 12, group 13, group 14 or group 15 of the periodic table or the like can be used. Of these metal salts, a metal salt such as yttrium nitrate is preferable in view of the high yield. By conducting the reaction using the acid in an amount of 0.01 to 2 equivalents based on the substrates, the target material can be obtained with a high yield. The reaction substrate (12) is generally used in an amount of 1 to 10 equivalents based on the substrate (6).

A solvent which does not adversely affect the reaction can be used as the solvent used in the reaction, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

[Production Method 10]

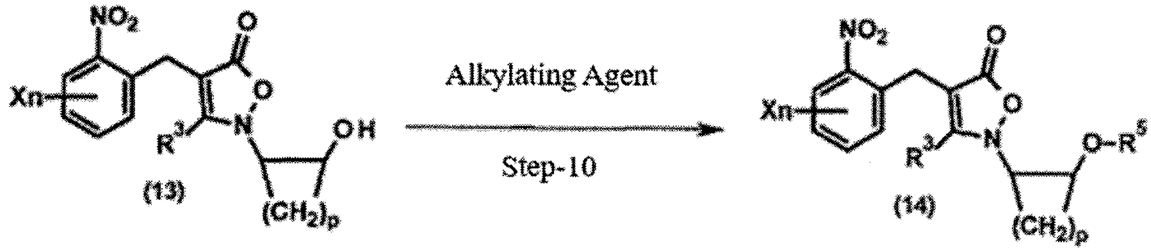


($R^3$, X, n and p have the same meanings as those described above. $R^5$ represents a C1-C6 alkyl group.)

The step-10 is a step of reacting an alkylating agent with the isoxazolin-5-one derivative represented by formula (13) to introduce $R^5$ to the hydroxyl group and thus producing an isoxazolin-5-one derivative represented by formula (14).

As the alkylating agent used in the reaction, an alkyl halide, an alkyl sulfonate, a sulfate ester, a trialkyloxonium tetrafluoroborate or the like can be used. Of these alkylating agents, a trialkyloxonium tetrafluoroborate is preferable in view of the high yield. The alkylating agent as a reaction substrate is generally used in an amount of 1 to 5 equivalents based on the substrate (13).

It is necessary to conduct the reaction in the presence of a base depending on the kind of the alkylating agent, and as the base, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine and 1,8-bis(dimethylamino)naphthalene, an alkali metal salt such as sodium carbonate, potassium carbonate, sodium hydrogen carbonate, potassium hydrogen carbonate, sodium acetate, potassium acetate, sodium methoxide, sodium ethoxide, potassium-tert-butoxide, sodium hydride, potassium hydride, sodium amide, butyllithium, tert-butyllithium, lithium diisopropylamide, trimethylsilyl lithium and lithium hexamethyldisilazide or the like can be used. Of these bases, an organic base such as 1,8-bis(dimethylamino)naphthalene is preferable in view of the high yield. By conducting the reaction using the base in an amount of 0.1 to 5 equivalents based on the substrates, the target material can be obtained with a high yield.

The reaction is preferably conducted in a solvent. A solvent which does not adversely affect the reaction can be used as the solvent, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentyl methyl ether, tetrahydrofuran, dimethoxyethane and 1,4-dioxane, a ketone such as

acetone, methyl ethyl ketone and cyclohexanone, a halogen-based solvent such as chloroform and dichloromethane, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethyl sulfoxide, water or a mixed solvent thereof can be used.

The reaction can be conducted at a temperature which is appropriately determined in the range of from -78°C to 200°C, although the temperature varies with the base used and the reaction conditions. After the reaction, the target material can be obtained by a general post-treatment operation, but the target material can also be purified by column chromatography, recrystallization or the like if necessary.

The compounds of the invention can be analyzed, confirmed or identified by the melting points, the infrared absorption spectra, $^{1}H$-NMR, $^{13}C$-NMR, mass spectrometry, X-ray structure analysis or the like, if necessary.

The production methods are not limited to those described above, and the compounds of the invention can be produced by any organic synthesis methods.

As also shown in Test Examples described below, the compounds of the invention exhibit an excellent herbicidal activity and exhibit an excellent selective weed killing activity distinguishing the weeds and the crops below. Thus, the compounds can be used for a wide range of targets such as weeds and the like in paddy rice fields and dry field. Specific examples of the weeds are as follows.

Specifically, for example, the following harmful weeds can be controlled: Gramineae weeds such as *Echinochloa crus-galli, Echinochloa oryzicola,* southern crabgrass *(Digitaria sanguinalis, Digitaria ischaem, Digitaria adscendens, Digitaria microbachne* or *Digitaria horizontalis), Setaria viridis, Setaria faberi, Setaria lutescens, Eleusine indica, Avena fatua, Sorghum halepense, Aropyron repens, Brachiaria plantaginea, Panicum maximum, Panicum purpurascens, Panicum dichotomiflorum, Leptochloa chinensis, Leptochloa panicea, Poa annua, Alopecurus aequalis, Alopecurus myosuroides, Agropyron tsukushiense, Brachiaria platyphylla, Cenchrus echinatus, Lolium multiflorum, Cynodon dactylon, Beckmannia syzigache, Bromus catharticus, Leersia japonica, Leersia sayanuka, Lolium rigidum, Paspalum distichum* and *Phleum pratense;* Cyperaceae weeds such as *Cyperus iria, Cyperus rotundus, Cyperus esculentus, Scirpus hotarui, Cyperus serotinus, Cyperus serotinus, Eleocharis acicularis, Eleocharis kuroguwai, Cyperus flaccidus, Kyllinga brevifolia* and *Scirpus juncoides;* Alismataceae weeds such as *Sagittaria pygmaea, Sagittaria trifolia* and *Alisma canaliculatum;* Pontderiaceae weeds such as *Monochoria vaginalis, Heteranthera limosa* and *Monochoria kosakowii;* Linderniaceae weeds such as *Lindernia pyxidaria;* Plantaginaceae weeds such as *Plantago asiatica, Gratiola japonica, Dopatrium junceum* and *Veronica polita;* Lythraceae weeds such as *Rotala india, Ammannia multifflora* and *Rotala indica;* Elatinaceae weeds such as *Elatine triandra;* Malvaceae weeds such as *Abutiol theophrsti* and *Sida spinosa;* Compositae weeds such as *Xanthium strumarim, Ambrosia elatior, Breea serosa, Galinsoga ciliata, Matricaria chamomilla, Taraxacum officinale, Erigeron canadensis, Bidens frondosa, Bidens pilosa, Bidens tripartita, Gnaphalium affine* and *Senecio vulgaris;* Lamiaceae weeds such as *Lamium amplexinale weber;* Solanaceae weeds such as *Solanum nigrum* and *Datura stramonium;* Amaranthaceae weeds such as *Amaranthus viridis, Chenopodium album, Kochia scoparia* and *Amaranthus hybridus;* Polygonaceeae weeds such as *Polygonum lapathifolium, Polygonum persicaria, Polygonum convolvulus, Polygonum aviculare, Persicaria longiseta* and *Persicaria nepalensis;* Crpurea weeds such as *Cardamine flexuosa, Capsella bursapastoris, Brassica juncea* and *Rorippa indica;* Convolvulaceae weeds such as *Ipomoea purpurea, Convolvulus arvensis, Ipomoea hederacea, Calystegia pubescens* and *Ipomoea coccinea;* Portulacaceae weeds such as *Portulaca oleracea;* Fabaceae weeds such as *Cassia obtusifolia, Aeschynomene indica, Sesbania exaltata, Trifolium repens* and *Vicia sativa;* Caryophyllaceae weeds such as *Stellaria media, Stellaria neglecta* and *Stellaria uliginosa;* Euphoribiaceae weeds such as *Euphorbia helioscopia* and *Acalypha australis;* Commelinaceae weeds such as *Commelina communis* and *Murdannia keisak;* Potamogetonaceae weeds such as *Potamogeton distinctus;* Araceae weeds such as *Spirodela polyrhiza;* Cucurbitaceae weeds such as *Sicyos angulatus;* Rubiaceae weeds such as *Galium spurium;* Apiaceae weeds such as *Oenanthejavanica;* Violaceae weeds such as *Viola mandshuria;* Onagraceae weeds such as *Ludwigia epilobioides* and *Oenothera odorata;* Oxalidaceae weeds such as *Oxalis corniculata;* Equisetaceae weeds such as *Equisetum arvense;* Zygnemataceae weeds such as *Spirogyra* sp. and the like. Accordingly, the compounds are effectively used for a case of selectively controlling a harmful weed or a case of non-selectively controlling a harmful weed in culturing, for example, *Zea mays, Glycine max, Gossypium* spp., *Triticum* spp., *Hordeum vulgare, Secale cereale, Avena sativa, Sorghum bicolor, Brassica napus, Helianthus annuus, Beta vulgaris, Saccharum officinarum, Zoysia japonicaa, Arachis hypogaea, Linum usitatissmum, Nicotiana tabacum, Coffea* spp. or the like, which are useful crops.

The applications of the herbicides of the invention are not limited to the weeds and the crops described above as examples.

If necessary, the compounds of the invention may be prepared as mixed formulations with another kind of herbicide, an insecticide, an acaricide, a nematicide, a germicide (a fungicide, a bactericide, an antiviral agent or a plant resistance inducer), a bird repellent, a plant growth regulator, a safener, a fertilizer, a soil conditioner, a synergist or the like during the formation or spraying or may be blended with such an agent in a tank mixer before spraying and applied.

In particular, when the compounds are blended and applied with another kind of herbicide, the amount of the used herbicide can be reduced, and the labor can be reduced. Moreover, a range of the targets of the herbicides (weed control spectrum) broadens due to a synergistic action of the agents, and a stronger effect can be expected due to a multiplier action of the agents. At this point, more than one kind of known herbicide or safener can also be combined and blended at the same time.

Of the optional components above, although representative examples of herbicides are shown below, the components are not limited to these examples only.

(1) Phenoxy-based compounds such as 2,4-D, 2,4-D-butotyl, 2,4-D-butyl, 2,4-D-dimethylammonium, 2,4-D-diolamine, 2,4-D-ethyl, 2,4-D-2-ethylhexyl, 2,4-D-isobutyl, 2,4-D-isoctyl, 2,4-D-isopropyl, 2,4-D-isopropylammonium, 2,4-D-sodium, 2,4-D-isopropanolammonium, 2,4-D-trolamine, 2,4-DB, 2,4-DB-butyl, 2,4-DB-dimethylammonium, 2,4-DB-isoctyl, 2,4-DB-potassium, 2,4-DB-sodium 2,4-D choline salt, dichlorprop, dichlorprop-butotyl, dichlorprop-dimethylammonium, dichlorprop-isoctyl, dichlorprop-potassium, dichlorprop-P, dichlorprop-P-dimethylammonium, dichlorprop-P-potassium, dichlorprop-P-sodium, MCPA, MCPA-butotyl, MCPA-dimethylammonium, MCPA-2-ethylhexyl, MCPA-potassium, MCPA-sodium, MCPA-thioethyl (MCAP-thioetyl), MCPB, MCPB-ethyl, MCPB-sodium, mecoprop, mecoprop-butotyl, mecoprop-sodium, mecoprop-P, mecoprop-P-butotyl, mecoprop-P-dimethylammonium, mecoprop-P-2-ethylhexyl, mecoprop-P-potassium, naproanilide, clomeprop and HIA-1; aromatic carboxylic acid-based compounds such as 2,3,6-TBA, dicamba, dicamba-butotyl, dicamba-diglycolamine, dicamba-dimethylammonium, dicamba-diolamine, dicamba-isopropylammonium, dicamba-potassium, dicamba-sodium, picloram, picloram-dimethylammonium, picloram-isooctyl, picloram-potassium, picloram-triisopropanolammonium, picloram-triisopropylammonium, picloram-trolamine, tricolopyr, tricolopyr-butotyl, tricolopyr-triethylammonium, clopyralid, clopyralid-olamine, clopyralid-potassium, clopyralid-triisopropanolammonium, aminopyralid, aminocyclopyrachlor, aminocyclopyrachlor, halauxifen, florpyrauxifen, halauxifen-methyl and DAS-534; and other compounds which are considered to exhibit a herbicidal efficacy by disturbing the hormone action of a plant, such as naptalam, naptalam-sodium, benazolin, benazolin-ethyl, quinclorac, quinmerac, diflufenzopyr, diflufenzopyr-sodium, fluroxypyr, fluroxypyr-2-butoxy-1-methylethyl, fluroxypyr-meptyl, chlorflurenol, chlorflurenol-methyl and clacyfos.

(2) Urea-based compounds such as chlorotoluron, diuron, fluometuron, linuron, isoproturon, metobenzuron, tebuthiuron, dimefuron, isouron, karbutilate, methabenztiazuron, metoxuron, metoburomuron, monolinuron, neburon, siduron, terbumeton and trietazine; triazine-based compounds such as simazine, atrazine, atratone, simetryn, prometryn, dimethametryn, hexazinone, metribuzin, terbuthylazine, cyanazine, ametryn, cybutryne, terbutryn, propazine, metamitron and prometon; uracil-based compounds such as bromacil, bromacyl-lithium, lenacil and terbacil; anilide-based compounds such as propanil and cypromid; carbamate-based compounds such as swep, desmedipham and phenmedipham; hydroxybenzonitrile-based compounds such as bromoxynil, bromoxynil-octanoate, bromoxynil-heptanoate, ioxynil, ioxynil-octanoate, ioxynil-potassium and ioxynil-sodium; and other compounds which are considered to exhibit a herbicidal efficacy by inhibiting the photosynthesis of a plant, such as pyridate, bentazone, bentazone-sodium, amicarbazone, methazole, pentanochlor and phenmedipham.

(3) Quaternary ammonium salt-based compounds which become free radicals in the plant and which are considered to generate active oxygen and exhibit an immediate herbicidal efficacy, such as paraquat and diquat.

(4) Diphenyl ether-based compounds such as nitrofen, chlomethoxyfen, bifenox, acifluorfen, acifluorfen-sodium, fomesafen, fomesafen-sodium, oxyfluorfen, lactofen, aclonifen, ethoxyfen-ethyl, fluoroglycofen-ethyl and fluoroglycofen; cyclic imide-based compounds such as chlorphthalim, flumioxazin, flumiclorac, flumiclorac-pentyl, cinidon-ethyl, fluthiacet-methyl and EK-5385; and other compounds which are considered to exhibit a herbicidal efficacy by inhibiting chlorophyll biosynthesis of a plant and causing abnormal accumulation of a photosensitizing peroxide substance in the plant, such as oxadiargyl, oxadiazon, sulfentrazone, carfentrazone-ethyl, thidiazimin, pentoxazone, azafenidin, isopropazole, pyraflufen-ethyl, benzfendizone, butafenacil, saflufenacil, fluazolate, profluazol, flufenpyr-ethyl, bencarbazone, tiafenacil, pyrachlonil, trifludimoxazin, HNPC-B4047, IR-6396, EK-5498, SYN-523 and the compound described in WO2008/008763 (FMC).

(5) Pyridazinone-based compounds such as norflurazon, chloridazon and metflurazon; pyrazole-based compounds such as pyrazolynate, pyrazoxyfen, benzofenap, topramezone, pyrasulfotole and tolpyralate; and other compounds which are considered to exhibit a herbicidal efficacy characterized by a bleaching effect by inhibiting biosynthesis of a pigment of a plant such as carotenoids, such as amitrole, fluridone, flurtamone, diflufenican, methoxyphenone, clomazone, sulcotrione, mesotrione, tembotrione, tefuryltrione, fenquinotrione, lancotrione, cyclopyrimorate, isoxaflutole, difenzoquat, difenzoquat-metilsulfate, isoxachlortole, benzobicyclon, bicyclopyron, picolinafen, beflubutamid, ketospiradox, ketospiradox-potassium and compounds described in JP2012/2571 (Sumitomo Chemical Company, Limited).

(6) Compounds which are considered to inhibit biosynthesis of fatty acids and exhibit a herbicidal efficacy on a plant including aryloxyphenoxypropionic acid-based compounds such as diclofop-methyl, diclofop, pyriphenop-sodium, fluazifop-butyl, fluazifop, fluazifop-P, fluazifop-P-butyl, haloxyfop, haloxyfop-etotyl, haloxyfop-P, haloxyfop-P-methyl,

quizalofop-ethyl, quizalofop-P, quizalofop-P-ethyl, quizalofop-P-tefuryl, cyhalofop-butyl, fenoxaprop-ethyl, fenoxaprop-P, fenoxaprop-P-ethyl, metamifop-propyl, metamifop, clodinafop-propargyl, propaquizafop, HNPC-A8169 and SYP-1924; cyclohexanedione-based compounds such as alloxydim-sodium, alloxydim, clethodim, sethoxydim, tralkoxydim, butroxydim, tepraloxydim, profoxydim and cycloxydim; phenylpyrazoline-based compounds such as pinoxaden; and the like.

(7) Sulfonylurea compounds such as chlorimuron-ethyl, chlorimuron, sulfometuron-methyl, sulfometuron, primisulfuron-methyl, primisulfuron, bensulfuron-methyl, bensulfuron, chlorsulfuron, metsulfuron-methyl, metsulfuron, cinosulfuron, pyrazosulfuron-ethyl, pyrazosulfuron, flazasulfuron, rimsulfuron, nicosulfuron, imazosulfuron, flucetosulfuron, cyclosulfamuron, prosulfuron, flupyrsulfuron-methyl-sodium, flupyrsulfuron, triflusulfuron, flupyrsulfuron-methyl-sodium, flupyrsulfuron, triflusulfuron-methyl, triflusulfuron, halosulfuron-methyl, halosulfuron, thifensulfuron-methyl, thifensulfuron, ethoxysulfuron, oxasulfuron, ethametsulfuron, ethametsulfuron-methyl, iodosulfuron, iodosulfuron-methyl-sodium, sulfosulfuron, triasulfuron, tribenuron-methyl, tribenuron, tritosulfuron, foramsulfuron, trifloxysulfuron, trifloxysulfuron-sodium, mesosulfuron-methyl, mesosulfuron, orthosulfamuron, amidosulfuron, azimsulfuron, propyrisulfuron, metazosulfuron, methiopyrsulfuron, monosulfuron-methyl, orsosulfuron, iofensulfuron and iofensulfuron-sodium; triazolopyrimidinesulfonamide-based compounds such as flumetsulam, metosulam, diclosulam, cloransulam-methyl, florasulam, penoxsulam and pyroxsulam; imidazolinone-based compounds such as imazapyr, imazapyr-isopropylammonium, imazethapyr, imazethapyr-ammonium, imazaquin, imazaquin-ammonium, imazamox, imazamox-ammonium, imazamethabenz, imazamethabenz-methyl and imazapic; pyrimidinyl salicylic acid-based compounds such as pyrithiobac-sodium, bispyribac-sodium, pyriminobac-methyl, pyribenzoxim, pyriftalid, pyrimisulfan and triafamone; sulfonylaminocarbonyltriazolinone-based compounds such as flucarbazone, flucarbazone-sodium, propoxycarbazone-sodium, propoxycarbazone and thiencarbazone-methyl; and other compounds which are considered to exhibit a herbicidal efficacy by inhibiting amino acid biosynthesis of a plant, such as glyphosate, glyphosate-sodium, glyphosate-potassium, glyphosate-ammonium, glyphosate-isopropylammonium, glyphosate-trimesium, glyphosate-sesquisodium, glufosinate, glufosinate-ammonium, glufosinate-P, glufosinate-P-ammonium, glufosinate-P-sodium, bilanafos, bilanafos-sodium and cinmethylin.

(8) Dinitroaniline-based compounds such as trifluralin, oryzalin, nitralin, pendimethalin, ethalfluralin, benfluralin, prodiamine, butralin and dinitramine; amide-based compounds such as bensulide, napropamide, napropamide-M, propyzamide and pronamide; organic phosphorus-based compounds such as amiprofos-methyl, butamifos, anilofos and piperophos; phenyl carbamate-based compounds such as propham, chlorpropham, barban and carbetamide; cumylamine-based compounds such as daimuron, cumyluron, bromobutide and methyldymron; and other compounds which are considered to exhibit a herbicidal efficacy by inhibiting cell mitosis of a plant, such as asulam, asulam-sodium, dithiopyr, thiazopyr, chlorthal-dimethyl, chlorthal, diphenamid, flamprop-M-methyl, flamprop-M and flamprop-M-isopropyl.

(9) Chloroacetamide-based compounds such as alachlor, metazachlor, butachlor, pretilachlor, metolachlor, S-metolachlor, thenylchlor, pethoxamid, acetochlor, propachlor, dimethenamide, dimethenamide-P, propisochlor and dimethachlor; thiocarbamate-based compounds such as molinate, dimepiperate, pyributicarb, EPTC, butylate, vernolate, cycloate, prosulfocarb, esprocarb, thiobencarb, diallate, tri-allate and orbencarb; and other compounds which are considered to exhibit a herbicidal efficacy by inhibiting protein biosynthesis or lipid biosynthesis of a plant, such as etobenzanid, mefenacet, flufenacet, tridiphane, cafenstrole, fentrazamide, ipfencarbazone, oxaziclomefone, indanofan, benfuresate, pyroxasulfone, fenoxasulfone, methiozolin, dalapon, dalapon-sodium, TCA-sodium and trichloracetic acid.

(10) Compounds which are considered to exhibit a herbicidal efficacy by inhibiting cellulose biosynthesis of a plant, such as dichlobenil, triaziflam, indaziflam, flupoxam and isoxaben.

(11) Other herbicides such as MSMA, DSMA, CMA, endothall, endothall-dipotassium, endothall-sodium, endothall-mono(N,N-dimethylalkylammonium), ethofumesate, sodium chlarate, pelargonic acid, nonanoic acid, fosamine, fosamine-ammonium, aclolein, ammonium sulfamate, borax, chloroacetic acid, sodium chloroacetate, cyanamide, methylarsonic acid, dimethylarsonic acid, sodium dimethylarsonate, dinoterb, dinoterb-ammonium, dinoterb-diolamine, dinoterb-acetate, DNOC, ferrous sulfate, flupropanate, flupropanate-sodium, mefluidide, mefluidide-diolamine, metam, metam-ammonium, metam-potassium, metam-sodium, methyl isothiocyanate, pentachlorophenol, sodium pentachlorophenoxide, pentachlorophenol laurate, quinoclamine, sulfuric acid, urea sulfate, zanthinosin, herbimycin, unguinol, metatyrosine, sarmentine, thaxtomin A, mevalocidin, alpha-limonene, pyribambenz-propyl, pyribambenz-isopropyl, JS-913, KHG-23844, H-9201, SIOC-0163, SIOC-0171, SIOC-0172, SIOC-0285, SIOC-0426, SIOC-H-057, ZJ-0166, ZJ-1835, ZJ-0453, ZJ-0777, ZJ-0862 and compounds described in WO2008/096398 (Kumiai Chemical Industry Co., Ltd.).

(12) Those which are considered to exhibit a herbicidal efficacy by parasitizing in a plant, such as *Xanthomonas campestris, Epicoccosirus nematosorus, Epicoccosirus nematosperus, Exserohilum monoseras* and *Drechsrela monoceras.*

When the compounds of the invention are used as herbicides, the compounds can be directly used but can also be used as formulations. To prepare the formulations, an appropriate carrier, an auxiliary agent, a surfactant, a binder, a stabilizer and the like described in Pesticide Formulation Guide (edited by Special Committee on Application of Pesticide Science Society of Japan, issued by Japan Plant Protection Association, 1997).

The herbicides containing the compounds of the invention can be formulated into any agent forms which are generally used as agent forms. For example, although the herbicides can be used in the forms of granules, microgranules, fine granules, water dispersible powder, a granulate water dispersible (dry flowable) agent, an emulsion, water soluble powder, a sol agent (flowable agent), a liquid, powder, rough powder, DL (driftless) powder, a flow dust agent, an oil, a microcapsule, a paste, a jumbo agent and the like, the forms are not limited to these examples.

As the carrier used for formulation, both solid and liquid can be used as long as the carrier is generally used for herbicide formulations. Although the carrier is not limited to a particular carrier, specific examples include the following carriers. Examples of the solid carrier include mineral powders (kaolin, bentonite, clay, montmorillonite, talc, diatomaceous earth, mica, vermiculite, quartz, calcium carbonate, apatite, white carbon, slaked lime, silica sand, Japanese acid clay, zeolite, sepiolite, expanded perlite powder, Shirasu-balloon, alumina balloon, a phenolic resin, an epoxy resin, microspheres of polyacrylonitrile, polyurethane or the like and the like), vegetable matter powders (soybean flour, wheat flour, wood flour, tobacco powder, starch, crystalline cellulose and the like), polymer compounds (a petroleum resin, polyvinyl chloride, a ketone resin and the like), alumina, silicate, glucose, sucrose, lactose, glycopolymers, ammonium sulfate, sodium chloride, potassium chloride, urea, highly dispersible silicic acid, waxes and the like.

Examples of the liquid carrier include water, alcohols (methyl alcohol, ethyl alcohol, n-propyl alcohol, isopropyl alcohol, butanol, ethylene glycol, benzyl alcohol and the like), aromatic hydrocarbons (toluene, benzene, xylene, ethyl benzene, methylnaphthalene and the like), ethers (ethyl ether, ethylene oxide, dioxane, tetrahydrofuran and the like), ketones (acetone, methyl ethyl ketone, cyclohexanone, methyl isobutyl ketone, isophorone and the like), esters (ethyl acetate, butyl acetate, ethylene glycol acetate, amyl acetate and the like), acid amides (dimethylformamide, dimethylacetamide and the like), nitriles (acetonitrile, propionitrile, acrylonitrile and the like), sulfoxides (dimethyl sulfoxide and the like), alcoholethers (ethylene glycol monomethyl ether, ethylene glycol monoethyl ether and the like), aliphatic or alicyclic hydrocarbons (n-hexane, cyclohexane and the like), industrial gasoline (petroleum ether, solvent naphtha and the like), petroleum fractions (paraffin, kerosene, light oil and the like) and the like.

When the herbicides are formulated into an emulsion, water dispersible powder, a flowable agent or the like, various kinds of surfactant are blended for the purpose of emulsification, dispersion, solubilization, wetting, foaming, lubrication, spreading or the like. Examples of such a surfactant include nonionic surfactants such as polyoxyethylene alkyl ethers, polyoxyethylene alkyl esters, polyoxyethylene sorbitan alkyl esters, polyoxyethylenealkyl aryl ethers, polyoxyethylene-polyoxypropylene block polymers and polyoxyethylene styrylphenylethers, anionic surfactants such as alkylbenzene sulfonates, alkyl sulfosuccinates, alkyl sulfates, polyoxyethylene alkyl sulfates, aryl sulfonates, alkylnaphthalene sulfonates, polyoxyethylene styrylphenylether sulfates, lignin sulfonates, naphthalene sulfonate formaldehyde condensate and polycarboxylates, cationic surfactants such as alkylamines (lauryl amine, stearyltrimethyl ammonium chloride and the like), polyoxyethylene alkyl amines, alkyl pyridinium salts, alkyltrimethyl ammonium salts and alkyldimethyl ammonium salts, ampholytic surfactants such as carboxylic acid (betaine type) and sulfate esters and the like, but the surfactant is not limited to the examples.

In addition, various kinds of auxiliary agents and additives such as polyvinyl alcohol (PVA), carboxymethyl cellulose (CMC), gum arabic, polyvinyl acetate, sodium alginate, gelatin, tragacanth gum, dextrin, hydroxypropyl methylcellulose (HPMC) and methyl cellulose (MC) and the like can be used.

An appropriate amount of the compound of the invention in the herbicide is around 0.01 to 90% based on the mass.

Preferable methods for using the herbicides containing the compounds of the invention as active ingredients include soil treatment, water surface treatment, leave and stem treatment and the like, and the herbicides can exhibit a particularly excellent effect when the herbicides are applied before germination and during the plumule period of a weed to be controlled.

Although the amount of the compound of the invention to be applied as a herbicide differs with the situation of the application, the time of the application, the application method, the target weed, the cultivated crop and the like, an appropriate amount of the active ingredient is generally around 0.001 to 10 Kg, and preferably around 0.01 to 1 Kg per hectare (ha).

## EXAMPLES

Although the invention is explained further specifically below using Synthesis Examples, Formulation Examples and Test Examples of the compounds of the invention, the invention is not limited to the examples.

In the Synthesis Examples and the Reference Examples below, the ratios described for the eluents or the mixed solvents indicate the volume ratios of the solvents.

[Synthesis Example 1]

Synthesis of 1,1,1-trifluoro-N-[2-[[(2-methoxycyclohexyl)-3-methyl-5-oxoisoxazol-4-yl]methyl]phenyl]methanesulfonamide (62) and 1,1,1-trifluoro-N-[2-[[(2-methoxycyclohexyl)-3-methyl-5-oxoisoxazol-4-yl]methyl]phenyl]-N-(trifluoromethylsulfonyl)methanesulfonamide (351)

Triethylamine (500 mg, 4.90 mmol) and trifluoromethanesulfonic anhydride (1.40 g, 4.90 mmol) were added at 0°C to a chloroform solution (50 ml) of 4-[(2-aminophenyl)methyl]-2-(2-methoxycyclohexyl)-3-methylisoxazol-5-one (1.20 g, 3.80 mmol), and the mixture was stirred at the same temperature for an hour. Water was poured into the reaction mixture, followed by extraction with chloroform. An extract was washed with saturated brine, dried over anhydrous sodium sulfate and then concentrated under reduced pressure. The concentrate was purified by silica gel column chromatography (eluent: ethyl acetate/n-hexane=1/2), and 1,1,1-trifluoro-N-[2-[[(2-methoxycyclohexyl)-3-methyl-5-oxoisoxazol-4-yl]methyl]phenyl]methanesulfonamide (amount of 400 mg, yield of 24%) as a yellow amorphous material and 1,1,1-trifluoro-N-[2-[[(2-methoxycyclohexyl)-3-methyl-5-oxoisoxazol-4-yl]methyl]phenyl]-N-(trifluoromethylsulfonyl)methanesulfonamide (amount of 400 mg, yield of 18%) as a yellow solid were thus obtained.

[Synthesis Example 2]

Synthesis of 1,1,1-trifluoro-N-[2-[[(2-methoxycyclopentyl)-3-methyl-5-oxoisoxazol-4-yl]methyl]phenyl]methanesulfonamide (59) and 1,1,1-trifluoro-N-[2-[[(2-methoxycyclopentyl)-3-methyl-5-oxoisoxazol-4-yl]methyl]phenyl]-N-(trifluoromethylsulfonyl)methanesulfonamide (349)

Triethylamine (1.74 g, 17.2 mmol) and trifluoromethanesulfonic anhydride (4.85 g, 17.2 mmol) were added at 0°C to a chloroform solution (50 ml) of 4-[(2-aminophenyl)methyl]-2-(2-methoxycyclopentyl)-3-methylisoxazol-5-one (4.00 g, 13.2 mmol), and the mixture was stirred at the same temperature for an hour. Water was poured into the reaction mixture, followed by extraction with chloroform. An extract was washed with saturated brine, dried over anhydrous sodium sulfate and then concentrated under reduced pressure. The concentrate was purified by silica gel column chromatography (eluent: ethyl acetate/n-hexane=1/2), and 1,1,1-trifluoro-N-[2-[[(2-methoxycyclopentyl)-3-methyl-5-oxoisoxazol-4-yl]methyl]phenyl]methanesulfonamide (amount of 4.50 g, yield of 78%) as a yellow oil and 1,1,1-trifluoro-N-[2-[[(2-methoxycyclopentyl)-3-methyl-5-oxoisoxazol-4-yl]methyl]phenyl]-N-(trifluoromethylsulfonyl)methanesulfonamide (amount of 400 mg, yield of 5%) as a yellow solid were thus obtained.

[Synthesis Example 3]

Synthesis of N-[2-[[(2-methoxycyclopentyl)-3-methyl-5-oxoisoxazol-4-yl]methyl]phenyl]-N-(trifluoromethylsulfonyl)acetamide (107)

Triethylamine (105 mg, 1.04 mmol) and acetyl chloride (65.1 mg, 0.829 mmol) were added at 0°C to a chloroform solution (5 ml) of 1,1,1-trifluoro-N-[2-[[(2-methoxycyclopentyl)-3-methyl-5-oxoisoxazol-4-yl]methyl]phenyl]methanesulfonamide (300 mg, 0.691 mmol), and the mixture was stirred at the same temperature for an hour. Water was poured into the reaction mixture, followed by extraction with chloroform. An extract was washed with saturated brine, dried over anhydrous sodium sulfate and then concentrated under reduced pressure. The concentrate was purified by silica gel column chromatography (eluent: ethyl acetate/n-hexane=1/2), ant the title compound (amount of 250 mg, yield of 78%) as a yellow oil was thus obtained.

[Synthesis Example 4]

Synthesis of ethyl N-[2-[[(2-methoxycyclohexyl)-3-methyl-5-oxoisoxazol-4-yl]methyl]phenyl]-N-(trifluoromethylsulfonyl)carbamate (190)

Sodium hydrogen carbonate (93.7 mg, 1.12 mmol) and ethyl chloroformate (121 mg, 1.12 mmol) were added to an acetonitrile solution (5 ml) of 1,1,1-trifluoro-N-[2-[[(2-methoxycyclohexyl)-3-methyl-5-oxoisoxazol-4-yl]methyl]phenyl]methanesulfonamide (250 mg, 0.557 mmol), and the mixture was heated under reflux for 5 hours. Water was poured into the reaction mixture, followed by extraction with ethyl acetate. An extract was washed with saturated brine, dried over anhydrous sodium sulfate and then concentrated under reduced pressure. The concentrate was purified by silica gel column chromatography (eluent: ethyl acetate/n-hexane=1/2), and the title compound (amount of 200 mg, yield of 69%) as a yellow oil was thus obtained.

The $^1$HNMR spectrum ($CDCl_3$) $\sigma$ (ppm) values, the melting points (°C) and the like of the compounds according

to the invention produced based on the above Synthesis Examples and the above production methods are shown in Table 2. The $^1$HNMR data were measured by JNM-ECS400 spectrometer (manufactured by JEOL Ltd.).

The compounds (288) and (289) in Table 2 below are isomers and are a compound of low polarity and a compound of high polarity, respectively.

[Table 2-1]

Table 2

| No. | Property (mp. °C) | Form | $^1$HNMR spectrum σ ppm: |
|---|---|---|---|
| 59 | | oil | 10.6(1H, br.s), 7.49(1H, d, J=8.0Hz), 7.28-7.14(3H, m), 4.14-4.08(1H, m), 3.73-3.68(1H, m), 3.58(2H, s), 3.10(3H, s), 2.35(3H, s), 2.01-1.89 (2H, m), 1.84-1.69 (3H, m), 1.59-1.50(1H, m). |
| 62 | | amorphous | 10.8(1H, br.s), 7.47(1H, d, J=8.4Hz), 7.22-7.18(1H, m), 7.14-7.13(2H, m), 3-62-3.50(3H, m), 3.16-3.09(1H, m), 2.91(3H, s), 2.30(3H, s), 2.18-2.14(1H, m), 1.95-1.74(3H, m), 1.27-1.02(4H, m). |
| 107 | | oil | 7.46-7.41(1H, m), 7.35(1H, t, J=7.6Hz), 7.30-7.20(2H, m), 4.17-4.09 (1H, m), 3.92-3.81(1H, m), 3.71-3.57(2H, m), 3.31(3H, d, J=14.8Hz), 2.21-2.17(6H, m), 2.09-1.80(2H, m), 1.80-1.71(2H, m), 1.65-1.54(2H, m). |
| 113 | | oil | 7.46-7.41(1H, m), 7.35(1H, t, J=7.6Hz), 7.29-7.22(2H, m), 4.17-4.09 (1H, m), 3.93-3.83(1H, m), 3.64(2H, s), 3.31(3H, d, J=12.0Hz), 2.57-2.49(1H, m), 2.16 (3H,d,J= 1.6Hz), 2.06-1.84(2H, m), 1.82-1.71 (2H, m), 1.65-1.56(2H, m), 1.20 (3H,d,J=6.8Hz),1.09(3H,d,J=8.0Hz). |
| 119 | | oil | 7.46-7.41(1H, m), 7.35(1H, t, J=7.2Hz), 7.30-7.18(2H, m), 4.17-4.09 (1H, m), 3.92-3.83(1H, m), 3.69-3.56(2H, m), 3.31(3H, d, J=14.4Hz), 2.35-2.17(2H, m), 2.17-1.87(4H, m), 1.81-1.72(2H, m), 1-65-1.56(2H, m), 1.65-1.56(2H, m), 1.08-1.03(1H, m), 1.00-0.88(6H, m). |
| 147 | | amorphous | 7.65-7.63(2H, m), 7.33-7.28(3H, m), 7.20-7.11(2H, m), 6.98-6.97(1H, m), 4.17-4.09(1H, m), 4.00-3.95(1H, m), 3.85-3.80(2H, m), 3-34(3H, d, J=24.8Hz), 2.24 (3H, d, J=2.0Hz), 2.09-1.86(2H, m), 1.82-1.71(2H, m), 1.64-1.56(2H, m). |
| 176 | | oil | 7.41-7.37(1H, m), 7.32-7.27(2H, m), 7.18(1H, d, J=8.0Hz), 4.38-4.30 (2H, m), 4.14-4.06(1H, m), 3.90-3.85(1H, m), 3.64(2H, s), 3.29(3H, s), 2.07(3H, d, J=5.2Hz), 2.03-1.69(5H, m), 1.63-1.57(1H, m), 1.32-1.24 (3H, m). |
| 188 | | oil | 7.41-7.37(1H, m), 7.32-7.27(2H, m), 7.18(1H, d, J=7.6Hz), 4.14-4.07 (1H, m), 3.90-3.84(4H, m), 3.64(2H, s), 3.29(3H, s), 2.08(3H, d, J=6.0Hz), 2.03-1.70(5H, m), 1.64-1.57(lH,m). |
| 190 | | oil | 7.39-7.28(3H, m), 7.17(1H, d, J=8.0Hz), 4.39-4.29(2H, m), 3-63(2H, s), 3.58-3.50 (1H, m), 3.38-3.29(1H, m), 3.22(3H, d, J=6.8Hz), 2.26-2.22(1H, m), 2, 08-2.02 (3H, m), 1.95-1. 77(3H, m), 1.34-1.05 (7H, m). |
| 200 | | oil | 7.41-7.37(1H, m), 7.32-7.28(2H, m), 7.18(1H, d, J=8.0Hz), 4.29-4.18 (2H, m), 4.14-4.06(1H, m), 3.90-3.85(1H, m), 3.65(2H, s), 3.29(3H, s), 2.07(3H, d, J=4.4Hz), 2.03-1.57(8H, m), 0.89-0.85(3H, m). |
| 212 | | oil | 7.40-7.36(1H, m), 7.31-7.27(2H, m), 7.17(1H, d, J=8.0Hz), 5.13-5.07 (1H, m), 4.15-4.06(1H, m), 3.91-3.86(1H, m), 3.65(2H, s), 3.29(3H, d, J=2.0Hz), 2.05 (3H, d, J=4.0Hz), 2.03-1.69(5H, m) 1.63-1.54( 1H, m), 1.34-1.29(6H,m). |

(continued)

| No. | Property (mp. °C) | Form | $^1$HNMR spectrum σ ppm: |
|---|---|---|---|
| 248 | | oil | 7.41-7.37(1H, m), 7.32-7.28(2H, m), 7.18(1H, d, J=7.2Hz), 4.14-4.06 (2H, m), 4.03-3.97(1H, m), 3.92-3.85(1H, m), 3.65(2H, s), 3.29(3H, s), 2.07(3H, d, J=4.0Hz), 2.03-1.69(6H, m) 1.63-1.56(1H, m), 0.88-0.83 (6H, m). |
| 250 | | oil | 7.39-7.27(3H, m), 7.18(1H, d, J=7.6Hz), 4.15-4.09(1H, m), 4.03-3.96 (1H, m), 3.64(2H, s), 3-57-3.49(1H, m), 3-37-3.30(1H, m), 3.22(3H, d, J=4.8Hz), 2.25-2.22(1H, m), 2.06-2.02(3H, m), 1.97-1.77(4H, m), 1.31-1.05(4H, m), 0.87-0.82(6H, m). |
| 286 | | oil | 7.44-7.24(7H, m), 7.21-7.19(2H, m), 4.10-4.05(1H, m), 3.89-3.84(1H, m), 3.77-3.75(2H, m), 3.27(3H, s), 2.08(3H, d, J=9.2Hz), 2.02-1.68 (5H, m), 1.62-1.55(1H, m). |
| 288 (TLC top) | | oil | 7.41-7.30(8H, m), 7.20(1H, d, J=8.0Hz), 3.75-3.73(2H, m), 3-58-3.50 (1H, m), 3.38-3.30(1H, m), 3.23-3.22(3H, m), 2.27-2.22(1H, m), 2.08-2.03(3H, m), 1.95-1.75(3H,m),1.29-1.15(4H,m). |

[Table 2-2]

| No. | Property (mp. °C) | Form | $^1$HNMR spectrum σ ppm: |
|---|---|---|---|
| 289 (TLC bottom) | | oil | 7.45-7.32(8H, m), 7.22(1H, d, J=7.6Hz), 3.74-3.72(2H, m), 3.57-3.50(1H, m), 3.38-3.31(1H, m), 3.24-3.23(3H, m), 2.27-2.22(1H, m), 2.10-2.02(3H, m), 1.99-1.77 (3H, m), 1.28-1.15(4H, m). |
| 349 | 103-105 | solid | 7.50-7.46(1H, m), 7.36-7.31(3H, m), 4.15-4.09(1H, m), 3.93-3.88(1H, m), 3.75 (2H, s), 3.31(3H, s), 2.06-2.01(2H, m), 2.03(3H, s), 1.97-1.86(2H, m), 1.82-1.73 (2H,m). |
| 351 | 103-105 | solid | 7.49-7.44(1H, m), 7.38-7.34(3H, m), 3.74(2H, s), 3.59-3.53(1H, m), 3.40-3.33 (1H, m), 3.25(3H, s), 2.28-2.25(1H, m), 2.00(3H, s), 2.00-1.78(3H, s), 1.34-1.06 (4H,m). |

Although the Reference Examples below show Synthesis Examples for synthesizing the starting substances of the syntheses above from commercial products, the syntheses are not limited to the examples.

[Reference Example 1]

Synthesis of 3-methyl-4-[(2-nitrophenyl)methyl]-2H-isoxazol-5-one

Ethyl acetoacetate (834 g, 641 mmol) was added at 0°C to a dimethoxyethane solution (1000 ml) of 60% sodium hydride (25.6 g, 641 mmol), and the mixture was stirred at room temperature (25°C) for 30 minutes. To the mixture solution, 2-nitrobenzyl chloride (100 g, 583 mmol) (manufactured by Tokyo Chemical Industry Co., Ltd.) was added at 0°C, and the mixture solution was stirred at 80°C for three hours. The reaction mixture was poured into an aqueous dilute hydrochloric acid solution, followed by extraction with ethyl acetate. The extract was washed with saturated brine, dried over anhydrous sodium sulfate and then concentrated under reduced pressure, and ethyl 2-[(2-nitrophenyl)methyl]-3-oxo-butanoate (amount of 155 g, yield of 100%) as a yellow oil was obtained. Hydroxylamine chloride (60.7 g, 873 mmol) was added to a methanol solution (500 ml) of the obtained oil, and the mixture was stirred at 80°C for an hour. Water was poured into the reaction mixture, followed by extraction with ethyl acetate. An extract was washed with saturated brine, dried over anhydrous sodium sulfate and then concentrated under reduced pressure. The concentrate was washed with a mixed solvent (ethyl acetate/n-hexane=1/2), and the title compound (amount of 100 g, yield of 73%) as a white solid was thus obtained.

Melting point: 148 to 150°C

$^1$HNMR spectrum (DMSO-d6) a: 12.1 (1H,br.s), 7.89 (1H,d,J=7.6Hz), 7.60 (1H,t,J=7.6Hz), 7.44 (1H,t,J=7.6Hz), 7.36 (1H,d,J=7.6Hz), 2.00 (3H,s).

[Reference Example 2]

Synthesis of 2-(2-hydroxycyclohexyl)-3-methyl-4-[(2-nitrophenyl)methyl]isoxazol-5-one

To 3-methyl-4-[(2-nitrophenyl)methyl]-2H-isoxazol-5-one (10.0 g, 42.7 mmol), 1,2-epoxycyclohexane (4.19 g, 42.7 mmol) and yttrium(III) nitrate hexahydrate (409 mg, 1.07 mmol) were added, and the mixture was stirred at room temperature for 10 hours. Water was poured into the reaction mixture, followed by extraction with ethyl acetate. An extract was washed with saturated brine, dried over anhydrous sodium sulfate and then concentrated under reduced pressure. The concentrate was washed with a mixed solvent (ethyl acetate/n-hexane=1/1), and the title compound (amount of 13.0 g, yield of 92%) as a white solid was thus obtained.

Melting point: 164 to 166°C

$^1$HNMR spectrum ($CDCl_3$) $\sigma$: 7.87 (1H,d,J=7.6Hz), 7.53-7.51 (2H,m), 7.38-7.34 (1H, m), 3.91-3.87 (3H,m), 3.49-3.47 (1H, m), 2.18-2.16 (4H,m), 2.09-2.07 (1H, m), 1.79-1.76 (4H,m), 1.32-1.27 (2H,m).

[Reference Example 3]

Synthesis of 2-(2-hydroxycyclopentyl)-3-methyl-4-[(2-nitrophenyl)methyl]isoxazol-5-one

The same reaction and treatment as those in Reference Example 2 were conducted using 1,2-epoxycyclopentane instead of 1,2-epoxycyclohexane, and the title compound (yield of 85%) as a white solid was obtained.

Melting point: 110 to 112°C

$^1$HNMR spectrum ($CDCl_3$) $\sigma$: 7.86 (1H, d, J=8.0Hz), 7.56-7.49 (2H,m), 7.39-7.35 (1H, m), 4.30-4.25 (1H, m), 4.07-4.02 (1H, m), 3.90 (2H,d,J=7.2Hz), 2.20 (3H,s), 2.05-1.71 (4H,m), 1.61-1.54 (2H,m).

[Reference Example 4]

Synthesis of 2-(2-methoxycyclohexyl)-3-methyl-4-[(2-nitrophenyl)methyl]isoxazol-5-one

To a dichloromethane solution (5 ml) of 2-(2-hydroxycyclohexyl)-3-methyl-4-[(2-nitrophenyl)methyl]isoxazol-5-one (5.00 g, 15.0 mmol), 1,8-bis(dimethylamino)naphthalene (9.67 g, 45.1 mmol) and trimethyloxonium tetrafluoroborate (4.45 g, 30.1 mmol) were added, and the mixture was stirred at room temperature for 3 hours. An aqueous 2N hydrochloric acid solution was added to the reaction mixture, and the precipitates were separated by filtration, followed by extraction with chloroform. An extract was washed with saturated brine, dried over anhydrous sodium sulfate and then concentrated under reduced pressure. The concentrate was purified by silica gel column chromatography (eluent: ethyl acetate/n-hexane=1/3), and the title compound (amount of 1.50 g, yield of 29%) as a yellow solid was obtained.

Melting point: 105 to 107°C

$^1$HNMR spectrum ($CDCl_3$) $\sigma$: 7.90 (1H,d,J=7.6Hz), 7.52-7.50 (2H,m), 7.37-7.33 (1H, m), 3.92 (2H,m), 3.56-3.50 (1H, m), 3.32-3.26 (1H, m), 3.15 (3H,s), 2.24-2.17 (1H, m), 2.13 (3H,s), 1.92-1.76 (4H,s), 1.30-1.08 (3H,m).

[Reference Example 5]

Synthesis of 2-(2-methoxycyclopentyl)-3-methyl-4-[(2-nitrophenyl)methyl]isoxazol-5-one

The same reaction and treatment as those in Reference Example 4 were conducted using 2-(2-hydroxycyclopentyl)-3-methyl-4-[(2-nitrophenyl)methyl]isoxazol-5-one instead of 2-(2-hydroxycyclohexyl)-3-methyl-4-[(2-nitrophenyl)methyl]isoxazol-5-one, and the title compound (yield of 45%) as a brown solid was thus obtained.

Melting point: 146 to 148°C

$^1$HNMR spectrum ($CDCl_3$) $\sigma$: 7.89 (1H, d, J=8.0Hz), 7.55-7.48 (2H,m), 7.39-7.35 (1H, m), 4.10-4.06 (1H, m), 3.92 (2H,s), 3.84-3.80 (1H, m), 3.27 (3H,s), 2.17 (3H,s), 2.04-1.88 (2H,m), 1.85-1.68 (2H,m), 1.61-1.53 (2H,m).

[Reference Example 6]

Synthesis of 4-[(2-aminophenyl)methyl] -2-(2-methoxycyclohexyl)-3 -methylisoxazol-5-one

Reduced iron (630 mg), ammonium chloride (600 mg, 11.0 mmol) and water (10 ml) were added to an ethanol solution (30 ml) of 2-(2-methoxycyclohexyl)-3-methyl-4-[(2-nitrophenyl)methyl]isoxazol-5-one (1.30 g, 3.80 mmol), and the mixture was stirred at 90°C for an hour. The reaction mixture was filtered through Celite, and an aqueous saturated sodium hydrogen carbonate solution was poured, followed by extraction with ethyl acetate. An extract was washed with

saturated brine, dried over anhydrous sodium sulfate and then concentrated under reduced pressure. The concentrate was purified by silica gel column chromatography (eluent: ethyl acetate/n-hexane=1/1), and the title compound (amount of 1.20 g, yield of 100%) as a yellow solid was obtained.

Melting point: 154 to 156°C

$^1$HNMR spectrum ($CDCl_3$) σ: 7.05-6.97 (2H,m), 6.65-6.60 (2H,m), 4.28 (2H, br.s), 3.51-3.39 (3H,m), 3.22-3.16 (1H, m), 2.99 (3H,s), 2.19-2.15 (4H,m), 1.94-1.73 (4H,m), 1.27-1.05 (3H,m).

[Reference Example 7]

Synthesis of 4-[(2-aminophenyl)methyl]-2-(2-methoxycyclopentyl)-3-methylisoxazol-5-one

The same reaction and treatment as those in Reference Example 6 were conducted using 2-(2-methoxycyclopentyl)-3-methyl-4-[(2-nitrophenyl)methyl]isoxazol-5-one instead of 2-(2-methoxycyclohexyl)-3-methyl-4-[(2-nitrophenyl)methyl]isoxazol-5-one, and the title compound (yield of 100%) as a brown solid was obtained.

Melting point: 93 to 95°C

$^1$HNMR spectrum ($CDCl_3$) σ: 7.04-7.00 (2H,m), 6.67-6.62 (2H,m), 4.22 (2H, br.s), 4.07-4.02 (1H, m), 3.79-3.74 (1H, m), 3.45 (2H,s), 3.19 (3H,s), 2.16 (3H,s), 2.01-1.66 (5H,m), 1.59-1.50 (1H, m).

Next, the methods for formulating the compounds of the invention as herbicides are explained specifically by the Formulation Examples below. In this regard, however, the herbicides are not limited to these Formulation Examples only and can be blended with various other additives at any ratios and formulated.

The "parts" in the Formulation Examples below represent the parts by mass, and "%" represents mass %.

[Formulation Example 1] (Granules)

Fifteen parts of water was added to 1 part of the compound of Synthesis Example 4, 1 part of calcium lignin sulfonate, 1 part of lauryl sulfate, 30 parts of bentonite and 67 parts of talc, and the mixture was kneaded with a kneader and then granulated with an extrusion granulator. By drying the granules with a fluidized-bed dryer, granules containing 1% active herbicide ingredient can be obtained. Furthermore, granules can be obtained by the same method except that each compound in Table 1 is used instead of the compound of Synthesis Example 1.

[Formulation Example 2] (Flowable Agent)

By evenly mixing and pulverizing 20.0 parts of the compound of Synthesis Example 4, 2.0 parts of di-2-ethylhexyl sulfosuccinate sodium salt, 2.0 parts of polyoxyethylene nonylphenyl ether, 5.0 parts of propylene glycol, 0.5 parts of a defoaming agent and 70.5 parts of water in a wet type ball mill, a flowable agent containing 20% active herbicide ingredient can be obtained. Furthermore, a flowable agent can be obtained by the same method except that each compound in Table 1 is used instead of the compound of Synthesis Example 1.

[Formulation Example 3] (Dry Flowable Agent)

By evenly mixing and finely pulverizing 75 parts of the compound of Synthesis Example 4, 10 parts of naphthalene sulfonate formaldehyde condensate, 5 parts of sodium lauryl sulfate, 5 parts of white carbon and 5 parts of clay, a dry flowable (granulate water dispersible) agent containing 75% active herbicide ingredient can be obtained. Furthermore, a dry flowable (granulate water dispersible) agent can be obtained by the same method except that each compound in Table 1 is used instead of the compound of Synthesis Example 1.

[Formulation Example 4] (Water Dispersible Powder)

By evenly mixing 15 parts of the compound of Synthesis Example 4, 15 parts of white carbon, 3 parts of calcium lignin sulfonate, 2 parts of polyoxyethylene alkyl ether, 5 parts of diatomaceous earth and 60 parts of clay with a pulverizing mixer, a water dispersible powder containing 15% active herbicide ingredient can be obtained. Furthermore, a water dispersible powder can be obtained by the same method except that each compound in Table 1 is used instead of the compound of Synthesis Example 1.

[Formulation Example 5] (Emulsion)

By mixing 20 parts of the compound of Synthesis Example 4, 18 parts of polyoxyethylene styrylphenylether, 2 parts of calcium dodecylbenzene sulfonate and 60 parts of xylene, an emulsion containing 20% active herbicide ingredient

can be obtained. Furthermore, an emulsion can be obtained by the same method except that each compound in Table 1 is used instead of the compound of Synthesis Example 1.

[Formulation Example 6] (Powder)

By evenly mixing and pulverizing 0.5 parts of the compound of Synthesis Example 4, 0.5 parts of white carbon, 0.5 parts of calcium stearate, 50.0 parts of clay and 48.5 parts of talc, a powder containing 0.5% active herbicide ingredient can be obtained. Furthermore, a powder can be obtained by the same method except that each compound in Table 1 is used instead of the compound of Synthesis Example 1.

[Formulation Example 7] (Jumbo Agent)

After mixing 15 parts of the compound of Synthesis Example 4, 2 parts of sodium lauryl sulfate, 5 parts of di-2-ethylhexyl sulfosuccinate sodium salt, 5 parts of carboxymethyl cellulose sodium salt, 35 parts of Shirasu-balloon, 10 parts of lactose and 28 parts of expanded perlite, 35 parts of water was added, and the mixture was kneaded with a kneader and then granulated with an extrusion granulator. By drying the granules with a fluidized-bed dryer, a jumbo agent containing 15% active herbicide ingredient can be obtained. Furthermore, a jumbo agent can be obtained by the same method except that each compound in Table 1 is used instead of the compound of Synthesis Example 1.

Next, Test Examples are shown in order to demonstrate the herbicidal effect of the isoxazolin-5-one derivatives of the invention.

<Test Example 1>

Herbicidal Effect Test by Treatment of Rice Paddy Soil

Wagner pots with an area of 1/10000 ares were filled with a paddy soil, and a compound fertilizer (N:P:K=17:17:17) was mixed after water was added, followed by soil puddling. Then, *Echinochloa crus-galli,* broad leaf weeds (*Lindernia pyxidaria* and *Monochoria vaginalis)* and *Scirpus juncoides,* 30 seeds each, were sown in a depth of 0 to 1 cm. Water was poured immediately after seeding, and the water depth was kept at about 3 cm. A subsequent management was conducted in a glass greenhouse. Immediately after that, emulsions prepared using the compounds in Table 3 below according to Formulation Example 5 were diluted with water, and a certain amount of the water-diluted agent solutions were dropped. A converted amount of the applied active ingredient corresponded to 120 g per 10 ares.

This test was conducted in a double system per one agent solution concentration area, and the herbicidal rates (%) were determined by the following equation (Math. 1) 14 days after the treatment with the agents.

[Math. 1]

$$\text{Herbicidal Rate (\%)} = \{1-(\text{Average Dry Weight (g) of Plant of Treated Area})/(\text{Average Dry Weight (g) of Plant of Untreated Area})\}\times 100$$

The results are shown in Table 3 below. In this regard, the compound numbers in Table 3 are the same as those in Table 1 and Table 2 above.

[Table 3]

Table 3

| No. | Concentration (g/10a) | *Echinochloa crus-galli* | *Monochoria vaginalis* | *Lindernia pyxidaria* | *Scirpus juncoides* |
|---|---|---|---|---|---|
| 59 | 120 | 100 | 100 | 100 | 100 |
| 62 | 120 | 100 | 100 | 100 | 100 |
| 107 | 120 | 100 | 100 | 100 | 100 |
| 113 | 120 | 100 | 100 | 100 | 100 |

(continued)

| No. | Concentration (g/10a) | *Echinochloa crus-galli* | *Monochoria vaginalis* | *Lindernia pyxidaria* | *Scirpus juncoides* |
|---|---|---|---|---|---|
| 119 | 120 | 100 | 100 | 100 | 100 |
| 147 | 120 | 100 | 100 | 100 | 100 |
| 176 | 120 | 100 | 100 | 100 | 100 |
| 188 | 120 | 100 | 100 | 100 | 100 |
| 190 | 120 | 100 | 100 | 100 | 100 |
| 200 | 120 | 100 | 100 | 100 | 100 |
| 212 | 120 | 100 | 100 | 100 | 100 |
| 248 | 120 | 100 | 100 | 100 | 100 |
| 250 | 120 | 100 | 100 | 100 | 100 |
| 286 | 120 | 100 | 100 | 100 | 100 |
| 288 (TLC top) | 120 | 100 | 100 | 100 | 100 |
| 289 (TLC bottom) | 120 | 100 | 100 | 100 | 100 |
| 349 | 120 | 100 | 100 | 100 | 90 |
| 351 | 120 | 100 | 100 | 100 | 90 |

<Test Example 2>

Herbicidal Effect Test by Treatment During Growing Period in Paddy Rice Cultivation

Wagner pots with an area of 1/10000 ares were filled with a paddy soil, and a compound fertilizer (N:P:K=17:17:17) was mixed after addition of water, followed by soil puddling. Then, *Echinochloa crus-galli,* broad leaf weeds (*Lindernia pyxidaria* and *Monochoria vaginalis)* and *Scirpus juncoides,* 30 seeds each, were sown in a depth of 0 to 1 cm. Water was poured immediately after seeding, and the water depth was kept at about 3 cm. A subsequent management was conducted in a glass greenhouse. Emulsions prepared using the compounds in Table 4 below according to Formulation Example 5 were diluted with water seven days after seeding, and a certain amount of the water-diluted agent solutions were dropped. A converted amount of the applied active ingredient corresponded to 120 g per 10 ares. The test was conducted in a double system per one agent solution concentration area, and the herbicidal rates (%) were determined by the equation (Math. 1) 14 days after the treatment with the agents. Results are shown in Table 4. In this regard, the compound numbers in Table 4 are the same as those in Table 1 and Table 2 above.

[Table 4]

Table 4

| No. | Concentration (g/10a) | *Echinochloa crus-galli* | *Monochoria vaginalis* | *Lindernia pyxidaria* | *Scirpus juncoides* |
|---|---|---|---|---|---|
| 59 | 120 | 100 | 90 | 80 | 90 |
| 62 | 120 | 100 | 90 | 80 | 90 |
| 107 | 120 | 100 | 90 | 80 | 90 |
| 113 | 120 | 100 | 90 | 80 | 90 |
| 119 | 120 | 100 | 90 | 80 | 90 |

(continued)

| No. | Concentration (g/10a) | *Echinochloa crus-galli* | *Monochoria vaginalis* | *Lindernia pyxidaria* | *Scirpus juncoides* |
|---|---|---|---|---|---|
| 147 | 120 | 100 | 90 | 80 | 90 |
| 176 | 120 | 100 | 90 | 80 | 90 |
| 188 | 120 | 100 | 90 | 80 | 90 |
| 190 | 120 | 100 | 90 | 80 | 90 |
| 200 | 120 | 100 | 90 | 80 | 90 |
| 212 | 120 | 100 | 90 | 80 | 90 |
| 248 | 120 | 100 | 90 | 80 | 90 |
| 250 | 120 | 100 | 90 | 90 | 90 |
| 286 | 120 | 100 | 90 | 80 | 90 |
| 288 (TLC top) | 120 | 100 | 90 | 90 | 90 |
| 289 (TLC bottom) | 120 | 100 | 90 | 90 | 90 |
| 349 | 120 | 100 | 80 | 80 | 90 |
| 351 | 120 | 100 | 80 | 80 | 90 |

<Test Example 3>

Herbicidal Effect Test by Treatment of Dry Field Farming Soil

Pots with a size of 36 $cm^2$ were filled with a dry field farming soil (alluvium). The soil of the top layer of 1 cm and seeds of weeds, namely southern crabgrass, *Echinochloa crus-galli, Chenopodium album* and *Amaranthus viridis,* 20 seeds each, were evenly mixed, and the top layer was gently pressed. Emulsions prepared using the compounds in Table 5 below according to Formulation Example 5 were diluted with water one day after seeding, and the water-diluted agent solutions were sprayed to the soil surfaces at a ratio of 100 liters per 10 ares. A converted amount of the applied active ingredient corresponded to 120 g per 10 ares. The herbicidal effects were evaluated by the same standard as that in Test Example 1, 14 days after the treatment with the agents. The results are shown in Table 5. In this regard, the compound numbers in Table 5 are the same as those in Table 1 and Table 2 above.

[Table 5]

Table 5

| No. | Concentration (g/10a) | southern crabgrass | *Echinochloa crus-galli* | *Chenopodium album* | *Amaranthus viridis* |
|---|---|---|---|---|---|
| 59 | 120 | 90 | 90 | 60 | 60 |
| 62 | 120 | 90 | 90 | 60 | 60 |
| 107 | 120 | 90 | 90 | 60 | 60 |
| 113 | 120 | 90 | 90 | 60 | 60 |
| 119 | 120 | 90 | 90 | 60 | 60 |
| 147 | 120 | 90 | 90 | 60 | 60 |
| 176 | 120 | 90 | 90 | 60 | 60 |
| 188 | 120 | 90 | 90 | 60 | 60 |

(continued)

| No. | Concentration (g/ 10a) | southern crabgrass | *Echinochloa crus-galli* | *Chenopodium album* | *Amaranthus viridis* |
|---|---|---|---|---|---|
| 190 | 120 | 90 | 90 | 60 | 90 |
| 200 | 120 | 90 | 90 | 60 | 60 |
| 212 | 120 | 90 | 90 | 60 | 60 |
| 248 | 120 | 90 | 90 | 60 | 60 |
| 250 | 120 | 90 | 90 | 60 | 90 |
| 286 | 120 | 90 | 90 | 60 | 60 |
| 288 (TLC top) | 120 | 90 | 90 | 60 | 40 |
| 289 (TLC bottom) | 120 | 90 | 90 | 60 | 40 |
| 349 | 120 | 90 | 90 | 60 | 90 |
| 351 | 120 | 90 | 90 | 60 | 90 |

<Test Example 4>

Herbicidal Effect Test by Treatment of Stem and Leaf in Dry Field Farming

Pots with a size of 36 $cm^2$ were filled with a dry field farming soil (alluvium). The soil of the top layer of 1 cm and seeds of weeds, namely southern crabgrass, *Echinochloa crus-galli, Chenopodium album* and *Amaranthus viridis,* 20 seeds each, were evenly mixed, and the top layer was gently pressed. Emulsions prepared using the compounds in Table 6 below according to Formulation Example 5 were diluted with water seven days after seeding, and the water-diluted agent solutions were sprayed to the soil surfaces at a ratio of 100 liters per 10 ares. A converted amount of the applied active ingredient corresponded to 120 g per 10 ares. The herbicidal effects were evaluated by the same standard as that in Test Example 1, 14 days after the treatment with the agents. The results are shown in Table 6. In this regard, the compound numbers in Table 6 are the same as those in Table 1 and Table 2 above.

[Table 6]

Table 6

| No. | Concentration (g/ 10a) | southern crabgrass | *Echinochloa crus-galli* | *Chenopodium album* | *Amaranthus viridis* |
|---|---|---|---|---|---|
| 59 | 120 | 90 | 90 | 90 | 90 |
| 62 | 120 | 90 | 90 | 90 | 90 |
| 107 | 120 | 80 | 90 | 90 | 90 |
| 113 | 120 | 80 | 90 | 90 | 90 |
| 119 | 120 | 80 | 90 | 90 | 90 |
| 147 | 120 | 80 | 90 | 90 | 90 |
| 176 | 120 | 80 | 90 | 90 | 90 |
| 188 | 120 | 80 | 90 | 90 | 90 |
| 190 | 120 | 90 | 90 | 90 | 90 |
| 200 | 120 | 80 | 90 | 90 | 90 |
| 212 | 120 | 80 | 90 | 90 | 90 |

(continued)

| No. | Concentration (g/ 10a) | southern crabgrass | *Echinochloa crus-galli* | *Chenopodium album* | *Amaranthus viridis* |
|---|---|---|---|---|---|
| 248 | 120 | 80 | 90 | 90 | 90 |
| 250 | 120 | 90 | 90 | 90 | 90 |
| 286 | 120 | 80 | 90 | 90 | 90 |
| 288 (TLC top) | 120 | 90 | 90 | 90 | 90 |
| 289 (TLC bottom) | 120 | 90 | 90 | 90 | 90 |
| 349 | 120 | 80 | 90 | 80 | 80 |
| 351 | 120 | 80 | 90 | 80 | 80 |

In Test Examples 1 to 4, a herbicidal rate of 80% or more is the maximum effect, and it has been confirmed that the effect is exhibited also in a test at a low concentration.

INDUSTRIAL APPLICABILITY

According to the invention, novel isoxazolin-5-one derivatives having an excellent herbicidal activity and herbicides containing the isoxazolin-5-one derivatives can be provided.

Although the invention has been explained in detail referring to specific embodiments, it is obvious to one skilled in the art that various changes and modifications can be made without departing from the spirit and the scope of the invention.

The application is based on a Japanese patent application filed on July 24, 2018 (patent application No. 2018-138495), which is hereby incorporated by reference.

## Claims

1. An isoxazolin-5-onederivative represented by formula (1) below:

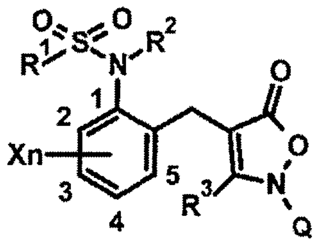

(1)

wherein $R^1$ represents a C1-C6 haloalkyl group,
$R^2$ represents a hydrogen atom, a C1-C6 alkyl group, a C1-C6 haloalkyl group, a C2-C6 alkenyl group, a C2-C6 alkynyl group, a C1-C6 alkoxy C1-C6 alkyl group, a C1-C6 haloalkoxy C1-C6 alkyl group, a C1-C6 alkoxy C1-C6 alkoxy C1-C6 alkyl group, a C1-C6 alkylthio C1-C6 alkyl group, a C1-C6 alkylcarbonyl C1-C6 alkyl group, a C7-C11 aralkyl group (the group may be monosubstituted or polysubstituted with a halogen atom(s), a C1-C6 alkyl group(s) or a C1-C6 alkoxy group(s)), a phenoxy C1-C6 alkyl group, a C7-C11 aralkyloxy C1-C6 alkyl group, a phenylcarbonyl C1-C6 alkyl group, a C1-C6 alkylcarbonyl group, a C1-C6 haloalkylcarbonyl group, a C2-C6 alkenylcarbonyl group, a C2-C6 alkynylcarbonyl group, a C3-C6 cycloalkylcarbonyl group, a C3-C6 cycloalkyl C1-C6 alkylcarbonyl group, a C1-C6 alkoxy C1-C6 alkylcarbonyl group, a C1-C6 haloalkoxy C1-C6 alkylcarbonyl group, a C1-C6 alkoxy C1-C6 alkoxy C1-C6 alkylcarbonyl group, a C1-C6 alkylthio C1-C6 alkylcarbonyl group, a C1-C6 haloalkylthio C1-C6 alkylcarbonyl group, a benzoyl group (the group may be monosubstituted or polysubstituted with a halogen atom(s) or a C1-C6 alkyl group(s)), a C7-C11 aralkylcarbonyl group (the group may be monosubstituted or polysubstituted

with a halogen atom(s) or a C1-C6 alkyl group(s)), a heterocyclic carbonyl group (the group may be monosubstituted or polysubstituted with a halogen atom(s) or a C1-C6 alkyl group(s)), a heterocyclic C1-C6 alkylcarbonyl group (the group may be monosubstituted or polysubstituted with a halogen atom(s) or a C1-C6 alkyl group(s)), a C1-C6 alkoxycarbonyl group, a C1-C6 haloalkoxycarbonyl group, a C2-C6 alkenyloxycarbonyl group, a C2-C6 alkynyloxycarbonyl group, a C3-C6 cycloalkyloxycarbonyl group, a C3-C6 cycloalkyl C1-C6 alkoxycarbonyl group, a C1-C6 alkoxy C1-C6 alkoxycarbonyl group, a C1-C6 haloalkoxy C1-C6 alkoxycarbonyl group, a C1-C6 alkoxy C1-C6 alkoxy C1-C6 alkoxycarbonyl group, a C1-C6 alkylthio C1-C6 alkoxycarbonyl group, a C1-C6 haloalkylthio C1-C6 alkoxycarbonyl group, a phenoxycarbonyl group (the group may be monosubstituted or polysubstituted with a halogen atom(s) or a C1-C6 alkyl group(s)), a C7-C11 aralkyloxycarbonyl group (the group may be monosubstituted or polysubstituted with a halogen atom(s) or a C1-C6 alkyl group(s)), a phenoxy C1-C6 alkoxycarbonyl group (the group may be monosubstituted or polysubstituted with a halogen atom(s) or a C1-C6 alkyl group(s)), a heterocyclic oxycarbonyl group (the group may be monosubstituted or polysubstituted with a halogen atom(s) or a C1-C6 alkyl group(s)), a heterocyclic C1-C6 alkoxycarbonyl group (the group may be monosubstituted or polysubstituted with a halogen atom(s) or a C1-C6 alkyl group(s)), a C1-C6 alkylthiocarbonyl group, a C1-C6 haloalkylthiocarbonyl group, a C1-C6 alkylaminocarbonyl group, a C1-C6 haloalkylaminocarbonyl group, a di-C1-C6 alkylaminocarbonyl group (the di-C1-C6 alkyl group moieties in the group may be the same or different), a C1-C6 alkylsulfonyl group, a C1-C6 haloalkylsulfonyl group, a C2-C6 alkenylsulfonyl group, a C2-C6 alkynylsulfonyl group, a C3-C6 cycloalkylsulfonyl group, a C3-C6 cycloalkyl C1-C6 alkylsulfonyl group, a C1-C6 alkoxy C1-C6 alkylsulfonyl group, a phenylsulfonyl group (the group maybe monosubstituted or polysubstituted with a halogen atom(s) or a C1-C6 alkyl group(s)), a C7-C11 aralkylsulfonyl group (the group may be monosubstituted or polysubstituted with a halogen atom(s) or a C1-C6 alkyl group(s)), a C1-C6 alkylaminosulfonyl group or a di-C1-C6 alkylaminosulfonyl group (the di-C1-C6 alkyl group moieties in the group may be the same or different),

$R^3$ represents a hydrogen atom, a halogen atom, a C1-C6 alkyl group, a C1-C6 haloalkyl group, a C3-C6 cycloalkyl group, a phenyl group (the group may be monosubstituted or polysubstituted with a halogen atom(s) or a C1-C6 alkyl group(s)), an amino group, a C1-C6 alkylamino group or a di-C1-C6 alkylamino group (the di-C1-C6 alkyl group moieties in the group may be the same or different),

Q represents a C3-C8 cycloalkyl group substituted with a C1-C6 alkoxy group,

X represents a hydrogen atom, a halogen atom, a C1-C6 alkyl group or a C1-C6 alkoxy group, and

n represents an integer of 1 to 4, wherein X's may be different from each other when n represents an integer of 2 to 4.

**2.** The isoxazolin-5-one derivative according to claim 1, wherein $R^1$ in formula (1) represents a C1-C6 fluoroalkyl group.

**3.** The isoxazolin-5-one derivative according to claim 1 or 2, wherein $R^1$ in formula (1) represents a trifluoromethyl group.

**4.** A herbicide comprising the isoxazolin-5-one derivative according to any one of claims 1 to 3 as an active ingredient.

## INTERNATIONAL SEARCH REPORT

International application No.
PCT/JP2019/028904

A. CLASSIFICATION OF SUBJECT MATTER
Int.Cl. C07D261/12(2006.01)i, A01N43/80(2006.01)i, A01P13/00(2006.01)i

According to International Patent Classification (IPC) or to both national classification and IPC

B. FIELDS SEARCHED

Minimum documentation searched (classification system followed by classification symbols)
Int.Cl. C07D261/12, A01N43/80, A01P13/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | SATO, K. et al., "Synthesis of 3-hydroxyisoxazoles from β-keto esters and hydroxylamine", Agricultural and Biological Chemistry, 1986, vol. 50, no. 7, pp. 1831-1837, ISSN 0002-1369, abstract, table 1 | 1-4 |
| A | JP 7-149742 A (RHONE POULENC AGRICULTURE LTD.) 13 June 1995, claims, examples & US 5489570 A, claims, examples & EP 636622 A1 & CN 1104212 A & KR 10-1995-0003278 A | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 August 2019 (27.08.2019) | 10 September 2019 (10.09.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/028904

C (Continuation). DOCUMENTS CONSIDERED TO BE RELEVANT

| Category* | Citation of document, with indication, where appropriate, of the relevant passages | Relevant to claim No. |
|---|---|---|
| A | JP 8-504781 A (RHONE POULENC AGRICULTURE LTD.) 21 May 1996, claims, examples & WO 1994/014782 A1, claims, examples & US 6323155 B1 & EP 674629 A1 & CN 1089258 A & KR 10-1995-0704274 A | 1-4 |
| A | JP 2015-500261 A (SYNGENTA LIMITED) 05 January 2015, claims, examples & WO 2013/083622 A1, claims, examples & US 2014/0329679 A1 & EP 2787815 A1 & CN 103974616 A & KR 10-2014-0101810 A | 1-4 |
| A | WO 2016/056565 A1 (ISHIHARA SANGYO KAISHA, LTD.) 14 April 2016, claims, examples & AR 102197 A1 & JP 2017-214292 A | 1-4 |
| A | US 3007936 A (HACO A.G.) 07 November 1961, example 23 (Family: none) | 1-4 |
| P, A | WO 2018/135649 A1 (HOKKO CHEMICAL INDUSTRY CO., LTD.) 26 July 2018, claims, examples (Family: none) | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4000155 A
- DE 3541722
- WO 2004011429 A
- WO 2006090792 A
- WO 2008059948 A
- WO 2008102908 A
- WO 2010026989 A
- WO 2010119906 A
- WO 2014175206 A
- WO 2016056565 A
- WO 2015004282 A
- WO 2015097071 A
- WO 2008008763 A
- JP 2012002571 A
- WO 2008096398 A
- JP 2018138495 A


### Non-patent literature cited in the description


- *Journal of Heterocyclic Chemistry,* 2013, vol. 50, 1381-1385